# FASCICULE DE BREVET EUROPEEN

(11) **EP 1 747 201 B1**
(45) Date de publication et mention de la délivrance du brevet: **15.10.2008**
(21) Numéro de dépôt: 05770962.8
(22) Date de dépôt: 10.05.2005
(51) Int. Cl.: C07D 231/54

(54) **DERIVES DE CARBAMATE DE 2H- OU 3H-BENZO[E]INDAZOL-1-YLE, LEUR PREPARATION ET LEUR APPLICATION EN THERAPEUTIQUE**
2H- ODER 3H-BENZO[E]INDAZOL-1-YL-KARBAMAT-DERIVATE, HERSTELLUNG UND THERAPEUTISCHE VERWENDUNG
2H OR 3H-BENZO[E]INDAZOL-1-YLE CARBAMATE DERIVATIVES, THE PREPARATION AND THERAPEUTIC USE THEREOF

(30) Priorité: 11.05.2004 FR 0405055
(43) Date de publication de la demande: 31.01.2007
(73) Titulaire: Sanofi-Aventis, 75013 Paris (FR)
(72) Inventeur: DUBOIS, Laurent, F-92350 Le Plessis-Robinson (FR); EVANNO, Yannick, 91490 Dannemois (FR); MALOIZEL, Christian, F-92190 Meudon (FR); SEVRIN, Mireille, F-75014 Paris (FR)
(74) Mandataire: Morel-Pécheux, Muriel
(86) Numéro de dépôt international: PCT/FR2005/001154
(87) Numéro de publication internationale: WO 2005/121099

(56) Documents cités:
- EP-A- 0 607 076
- EP-A- 1 036 794
- WO-A-99/58117
- FR-A- 2 741 073
- YOKOYAMA, NAOKATA ET AL: "2-Arylpyrazolo[4,3-c]quinolin-3-ones: a novel agonist, a partial agonist and an antagonist of benzodiazepines" JOURNAL OF MEDICINAL CHEMISTRY, vol. 25, no. 4, 1982, pages 337-339, XP002312656
- LLOYD G K ET AL: "THE ACTIVITY OF ZOLPIDEM AND OTHER HYPNOTICS WITHIN THE GAMMA-AMINOBUTYRIC ACID (GABAA) RECEPTOR SUPRAMOLECULAR COMPLEX, AS DETERMINED BY 35S-T-BUTYLBICYCLOPHOPHOROTHIONATE (35S-TBPS) BINDING TO RAT CEREBRAL CORTEX MEMBRANES" JOURNAL OF PHARMACOLOGY AND EXPERIMENTAL THERAPEUTICS, AMERICAN SOCIETY FOR PHARMACOLOGY AND, US, vol. 255, no. 2, octobre 1990 (1990-10), pages 690-696, XP008001863 ISSN: 0022-3565 & WEISSMAN, BEN A. ET AL: "Presence of peripheral benzodiazepine binding sites on primary rat skeletal fibroblasts" EUROPEAN JOURNAL OF PHARMACOLOGY, vol. 187, no. 3, 1990, pages 369-375, XP002312657
- NAKAO, TOHRU ET AL: "Synthesis and biological activities of optical isomers of 2-(4-chlorophenyl)-5,6-dihydro-(1)benzothi epino[5,4-c]pyridazin- 3(2H)-one 7-oxide" CHEMICAL & PHARMACEUTICAL BULLETIN ( 1992 ), 40 (1), 117 -21 CODEN: CPBTAL; ISSN: 0009-2363, vol. 40, no. 1, 1992, pages 117-121, XP002312658
- PIPERAKI S ET AL: "Enantiomeric separation of zopiclone, its metabolites and products of degradation on a beta-cyclodextrin bonded phase" JOURNAL OF CHROMATOGRAPHY A, ELSEVIER SCIENCE, NL, vol. 729, no. 1, 5 avril 1996 (1996-04-05), pages 19-28, XP004039158 ISSN: 0021-9673
- OLIVIER A ET AL: "A conformational study of ligands for omega modulatory sites of GABAA receptors by noesy NMR spectroscopy and distance geometry" BIOORGANIC & MEDICINAL CHEMISTRY LETTERS, OXFORD, GB, vol. 7, no. 17, 9 septembre 1997 (1997-09-09), pages 2277-2282, XP004136428 ISSN: 0960-894X
- RAO, V. L. RAGHAVENDRA ET AL: "Characterization of binding sites for the .omega.3 receptor ligands [3H]PK11195 and [3H]RO5-4864 in human brain" EUROPEAN JOURNAL OF PHARMACOLOGY, vol. 340, no. 1, 1997, pages 89-99, XP002312659

## Description

Dérivés de carbamate de 2*H*- ou 3*H*-benzo[e]indazol-1-yle, leur préparation et leur application en thérapeutique

L'invention a pour objet des composés dérivés de carbamate de 2*H*- ou 3*H-*benzo[e]indazol-1-yle qui présentent une affinité *in vitro* et *in vivo* pour les récepteurs de type périphérique aux benzodiazépines (sites p ou PBR).

Des composés présentant une affinité pour les PBR sont connus de l'art antérieur, par exemple des dérivés d'isoquinolines (WO99/58117 ; Lloyd et al. 1990 J. of Pharm and Experimental Therapeutics.), d'imidazo-benzimidazoles (EP0607076), d'imidazo-pyrrolobenzimidazoles (FR2741073), de 2-aryl-8-oxodihydropurines (EP1036794), de 2-arylpyrazolo-quinolinones (Yokoyama et al. 1982 J. of Med. Chem.), d'imidazopyridine et pyrrolopyrazine (Weissman et al. 1990 Eur. J. of Pharmacol. ; Piperaki et al. 1996 J. of Chromatography; Olivier et al. 1997 Bioorg. and Med. Chem Letters), des dérivés de benzothieno-pyridazin-3-one-7-oxide (Nakao et al. 1992 Chem, and Pharm. Bull.), ou d'indoleacétamide (Rao et al. 1997 Eur. J. of Pharmacol.).
Néanmoins, il existe toujours un besoin de trouver des composés affins pour les PBR.

Un premier objet de l'invention concerne les composés répondant à la formule générale (I) ci-après.
Un autre objet de l'invention concerne des procédés de préparation des composés de formule générale (I).
Un autre objet de l'invention concerne les utilisations des composés de formule générale (I) notamment dans des médicaments ou dans des compositions pharmaceutiques.

Les composés de l'invention répondent à la formule générale (I) ; 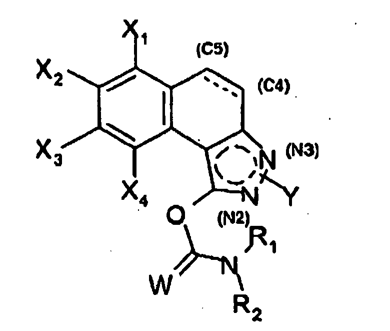 dans laquelle
W représente un atome d'oxygène ou de soufre ;
X₁, X₂, X₃ et X₄ représentent chacun, indépendamment l'un de l'autre, un atome d'hydrogène ou d'halogène ou un groupe cyano, C₁-C₆-alkyle, C₁-C₆-fluoroalkyle, C₁-C₆-alcoxy, C₁-C₆-fluoroalcoxy ;
Y est en position (N2) ou (N3) ;
quand Y est en position (N2), Y représente un groupe C₁-C₆-alkyle, C₁-C₆-fluoroalkyle, aryle, comprenant entre 6 et 10 atomes de carbone, ou hétéroaryle, comprenant de 5 à 6 chaînons et comprenant entre 1 et 2 hétéroatomes ;
quand Y est en position (N3), Y représente un groupe aryle ou hétéroaryle ;
les groupes aryle ou hétéroaryle étant éventuellement substitués par un ou plusieurs atomes ou groupes choisis parmi les atomes d'halogène, les groupes C₁-C₆-alkyle, C₁-C₆-alcoxy, C₁-C₆-alkylthio, C₁-C₆-alkyl-S(O)-, C₁-C₆-alkyl-S(O)₂-, C₁-C₆-fluoroalkyle ; la liaison en position C4-C5 est double ou simple ;
R₁ et R₂ représentent chacun, indépendamment l'un de l'autre, un groupe aryle, comprenant entre 6 et 10 atomes de carbone, benzyle ou C₁-C₆-alkyle ; ou bien R₁ et R₂ forment, avec l'atome d'azote qui les porte un hétérocycle, comprenant de 4 à 7 chaînons et comprenant un atome d'azote et éventuellement un autre hétéroatome, éventuellement substitué par un ou plusieurs groupes C₁-C₆-alkyle ou benzyle.

Dans le cadre de la présente invention on entend par :
- C₁-C₂ où t et z peuvent prendre les valeurs de 1 à 6, une chaîne carbonée pouvant avoir de t à z atomes de carbone, par exemple C₁₋₃ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un alkylthio : un radical -S-alkyle où le groupe alkyle est tel que précédemment défini ;
- un hétérocycle : un groupe cyclique comprenant de 4 à 7 chaînons et comprenant un atome d'azote et éventuellement un autre hétéroatome, tel que l'azote, l'oxygène ou le soufre. A titre d'exemples d'hétérocycles, on peut citer les groupes azétidinyle, pyrrolidinyle, pipéridinyle, morpholinyle, thiomorpholinyle, azépinyle, pipérazinyle ou homopipérazinyle ;
- un aryle : un groupe aromatique cyclique comprenant entre 6 et 10 atomes de carbone. A titre d'exemples de groupes aryles, on peut citer les groupes phényle ou naphtyle ;
- un hétéroaryle : un groupe aromatique cyclique comprenant de 5 à 6 chaînons et comprenant entre 1 et 2 hétéroatomes, tels que l'azote, l'oxygène ou le soufre. A titre d'exemples de groupes hétéroaryles, on peut citer les groupes pyridinyle, thiényle, furanyle, pyrimidinyle, pyrazinyle ou pyridazinyle ;
- un chainon : dans un groupe cyclique, représente une liaison entre deux atomes adjacents ;
- un atome d'halogène : un fluor, un chlore; un brome ou un iode.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvates, à savoir sous forme d'associations ou de combinaisons avec une ou plusieurs molécules d'eau ou avec un solvant. De tels hydrates et solvates font également partie de l'invention.

Parmi les composés de formule (I) objets de l'invention, un premier sous-groupe de composés est constitué par les composés pour lesquels :
W représente un atome d'oxygène ou de soufre ; et/ou
X₁, X₂ et X₃ représentent chacun, indépendamment l'un de l'autre, un atome d'hydrogène ou d'halogène, plus particulièrement de fluor, de chlore ou de brome, ou un groupe cyano, C₁-C₆-alkyle, plus particulièrement méthyle, C₁-C₆-alcoxy, plus particulièrement méthoxy ; et/ou
X₄ représente un atome d'hydrogène ; et/ou
Y est en position (N2) ou (N3) ;
quand Y est en position (N2), Y représente un groupe C₁-C₆-alkyle, plus particulièrement méthyle ou éthyle, C₁-C₆-fluoroalkyle, plus particulièrement trifluoroéthyle, aryle, comprenant entre 6 et 10 atomes de carbone, plus particulièrement phényle, ou hétéroaryle, comprenant de 5 à 6 chaînons et comprenant entre 1 et 2 hétéroatomes, plus particulièrement pyridinyle ou pyrazinyle ;
quand Y est en position (N3), Y représente un groupe aryle, plus particulièrement phényle, ou hétéroaryle, plus particulièrement pyridinyle ou pyrimidinyle ;
les groupes aryle ou hétéroaryle étant éventuellement substitués par un ou plusieurs atomes ou groupes, plus particulièrement par un ou deux atomes ou groupes, choisis parmi les atomes d'halogène, plus particulièrement de fluor, de chlore, les groupes C₁-C₆-alkyle, plus particulièrement méthyle, et C₁-C₆-alcoxy, plus particulièrement méthoxy;
   et/ou
la liaison en position C4-C5 est double ou simple ; et/ou
R₁ et R₂ représentent chacun, indépendamment l'un de l'autre, un groupe aryle, comprenant entre 6 et 10 atomes de carbone, plus particulièrement phényle, C₁-C₆-alkyle, plus particulièrement méthyle, éthyle, n-propyle, t-butyle, isopropyle ; ou bien R₁ et R₂ forment, avec l'atome d'azote qui les porte, un hétérocycle, comprenant de 4 à 7 chaînons et comprenant un atome d'azote et éventuellement un autre hétéroatome, plus particulièrement pyrrolidinyle, pipéridinyle, morpholinyle ou pipérazinyle, éventuellement substitué par un ou deux groupes C₁-C₆-alkyle, plus particulièrement méthyle.

Parmi les composés de formule (I) objets de l'invention, un second sous-groupe de composés est constitué par les composés pour lesquels :
W représente un atome d'oxygène ou de soufre ; et/ou
X₁, X₂ et X₃ représentent chacun, indépendamment l'un de l'autre, un atome d'hydrogène ou d'halogène, plus particulièrement de fluor, de chlore ou de brome, ou un groupe C₁-C₆-alkyle, plus particulièrement méthyle, C₁-C₆-alcoxy, plus particulièrement méthoxy;
   et/ou
X₄ représente un atome d'hydrogène ; et/ou
Y est en position (N2) ou (N3) et représente un groupe aryle, , comprenant entre 6 et 10 atomes de carbone, plus particulièrement phényle, ou hétéroaryle, comprenant de 5 à 6 chaînons et comprenant entre 1 et 2 hétéroatomes, plus particulièrement pyridinyle, pyrazinyle ou pyrimidinyle ;
les groupes aryle ou hétéroaryle étant éventuellement substitués par un ou plusieurs atomes ou groupes, plus particulièrement par un ou deux atomes ou groupes, choisis parmi les atomes d'halogène, plus particulièrement de fluor, de chlore, les groupes C₁-C₆-alkyle, plus particulièrement méthyle, et C₁-C₆-alcoxy, plus particulièrement méthoxy ;
   et/ou
la liaison en position C4-C5 est double ou simple ; et/ou
R₁ et R₂ représentent chacun, indépendamment l'un de l'autre, un groupe aryle, comprenant entre 6 et 10 atomes de carbone, plus particulièrement phényle, C₁-C₆-alkyle, plus particulièrement méthyle, éthyle, n-propyle, t-butyle, isopropyle ; ou bien R₁ et R₂ forment, avec l'atome d'azote qui les porte, un hétérocycle, comprenant de 4 à 7 chaînons et comprenant un atome d'azote et éventuellement un autre hétéroatome, plus particulièrement pyrrolidinyle, pipéridinyle, morpholinyle ou pipérazinyle, éventuellement substitué par un ou deux groupes C₁-C₆-alkyle, plus particulièrement méthyle.

Parmi les composés de formule (I) objets de l'invention, un troisième sous-groupe de composés est constitué par les composés pour lesquels :
W représente un atome d'oxygène ou de soufre ; et/ou
X₁, X₂ et X₃ représentent chacun, indépendamment l'un de l'autre, un atome d'hydrogène ou d'halogène, plus particulièrement de fluor, de chlore ou de brome, ou un groupe C₁-C₆-alkyle, plus particulièrement méthyle, C₁-C₆-alcoxy, plus particulièrement méthoxy ;
   et/ou
X₄ représente un atome d'hydrogène ; et/ou
Y est en position (N3) et représente un groupe aryle, ; comprenant entre 6 et 10 atomes de carbone, plus particulièrement phényle, ou hétéroaryle, comprenant de 5 à 6 chaînons et comprenant entre 1 et 2 hétéroatomes, plus particulièrement pyridinyle ou pyrimidinyle ;
les groupes aryle ou hétéroaryle étant éventuellement substitués par un ou plusieurs atomes ou groupes, plus particulièrement par un ou deux atomes ou groupes, choisis parmi les atomes d'halogène, plus particulièrement de fluor, de chlore, les groupes C₁-C₆-alkyle, plus particulièrement méthyle, et C₁-C₆-alcoxy, plus particulièrement méthoxy ;
   et/ou
la liaison en position C4-C5 est double ou simple ; et/ou
R₁ et R₂ représentent chacun, indépendamment l'un de l'autre, un groupe aryle, comprenant entre 6 et 10 atomes de carbone, plus particulièrement phényle, C₁-C₆-alkyle, plus particulièrement méthyle, éthyle, t-butyle, isopropyle ; ou bien R₁ et R₂ forment, avec l'atome d'azote qui les porte, un hétérocycle, comprenant de 4 à 7 chaînons et comprenant un atome d'azote et éventuellement un autre hétéroatome, plus particulièrement pipéridinyle, éventuellement substitué par un ou deux groupes C₁-C₆-alkyle, plus particulièrement méthyle.

Les composés de formule générale (I), peuvent être préparés par les procédés illustrés dans les schémas suivants.

Selon une première voie de préparation (schéma 1), un composé de formule générale (II), dans laquelle X₁, X₂, à et X₄ sont tels que définis dans la formule générales (I), est mis en réaction avec du carbonate de méthyle en présence d'une quantité catalytique d'une base telle que du méthanolate de sodium ou de l'hydrure de sodium pour obtenir le cétoester de formule générale (III). La condensation du cétoester (III) avec de l'hydrazlne, par exemple dans un solvant polaire tel que le DMF ou l'acide acétique, permet d'isoler le pyrazole de formule générales (IV). Celui-ci est ensuite N-substitué non sélectivement par action d'un halogénure d'aryle ou d'hétéroaryle de formule générale Y-hal, dans laquelle Y est tel que défini dans la formule générale (I) et hal est un atome d'halogène, tel qu'un iode ou un brome, en présence d'une base telle que du carbonate de potassium ou de césium ou de triphosphate de potassium, d'une quantité catalytique d'un sel de cuivre et d'une diamine (S.L. Buchwald, J. Am. Chem. Soc., 2001, 123, 7727).

Le mélange résultant constitué des isomères de position de formule générale (Va) et (Vb), dans lesquelles le groupe Y est respectivement en position 2 et 3 du cycle pyrazole, est ensuite dérivatisé par action d'un dérivé de chlorure de carbamoyle de formule générale CIC(W)NR₁R₂, dans laquelle W, R₁ et R₂ sont tels que définis dans la formule générale (I), en présence d'une base telle que le carbonate de potassium, l'hydrure de sodium ou la triéthylamine pour obtenir les carbamates de formules générales (Ia) et (Ib) qui sont séparés, à cette étape, par les méthodes connues de l'homme de l'art, telle que la chromatographie sur colonne de silice.

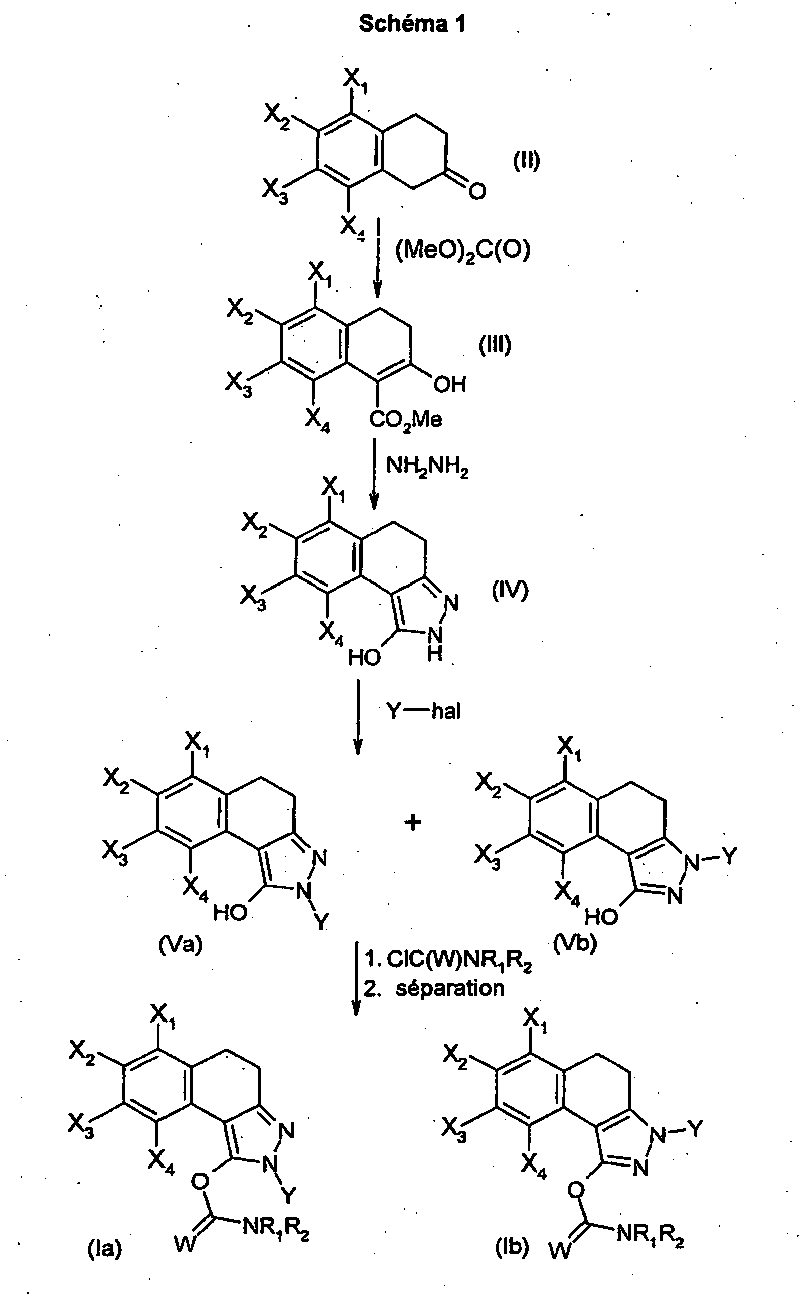

Alternativement, une seconde voie de préparation permet de préparer les composés de formule générale (la) (schéma 2).

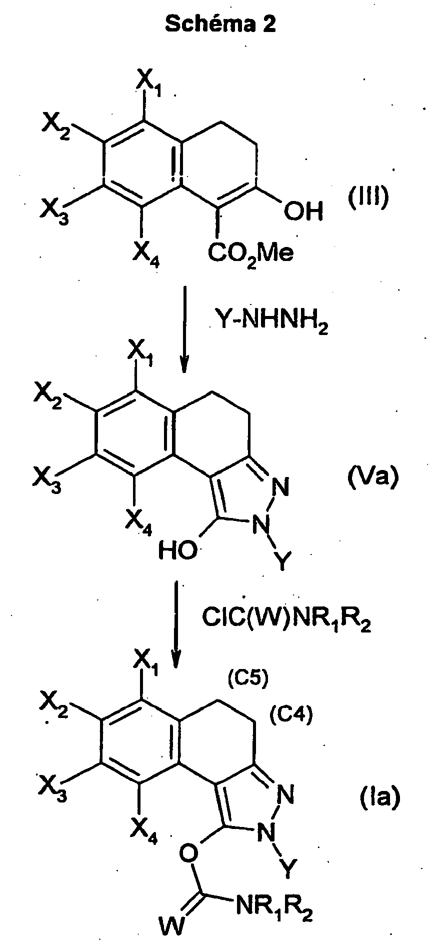

Elle consiste à condenser le cétoester de formule générale (III) telle que définie ci-dessus, avec une hydrazine de formule générale Y-NH-NH₂, dans laquelle Y est tel que défini dans la.formule générale (I), par exemple dans un solvant polaire tel que le DMF ou l'acide acétique, et permet d'isoler le pyrazole de formule générale (Va), telle que définie ci-dessus. Ce dernier est ensuite acylé par action d'un dérivé de chlorure de carbamoyle de formule générale CIC(W)NR₁R₂, telle que définie ci-dessus, en présence d'une base telle que le carbonate de potassium, l'hydrure de sodium ou la triéthylamine pour obtenir le carbamate de formule générale (Ia).

La liaison simple en position C4-C5 des composés de formule générale (I) peut éventuellement être déhydrogénée pour former une liaison double selon une méthode connue par l'homme de l'art, par exemple par analogie à la méthode décrite par Kozo, Shishido et al. Tetrahedron. 1989, 45, 18, 5791-5804. Alternativement, les composés de formule générale (I) comportant une liaison simple en position C4-C5 peuvent être déhydrogénés par réaction avec un agent halogénant, tel que le N-bromosuccinimide en présence d'un initiateur tel que le 2,2'-azobis(2-méthylpropionitrile). Dans ces conditions, le composé de formule générale (I) comportant une liaison simple en position C4-C5 est d'abord halogéné, puis l'intermédiaire résultant subit une réaction d'élimination pour conduire au composé (I) comportant une double liaison en position C4-C5.

Dans les schémas 1 et 2, les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.
Les composés de formule générale (II) peuvent être obtenus de sources commerciales ou préparés en utilisant des méthodes décrites dans la littérature (Sims, J.J. et al. Tetrahedron Lett. 1971, 951).

L'invention, selon un autre de ses aspects, a également pour objet les composés de formules (Va) et (Vb). Ces composés peuvent être utiles comme intermédiaires de synthèse des composés de formule (I).
Le tableau 1 qui suit illustre les structures chimiques et les propriétés physiques de quelques composés de formules générales (Va) et (Vb) de l'invention. La colonne "PF" renseigne les points de fusion des produits.

**Tableau 1**

| | | | | | | |
|---|---|---|---|---|---|---|
| 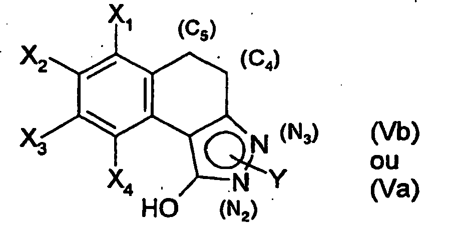 | | | | | | |

| **n°** | **X**₁ | **X**₂ | **X**₃ | **X**₄ | **Y** | **PF (°C)** |
|---|---|---|---|---|---|---|
| Va.1 | H | H | H | H | 2-(4-méthylphényl) | 221-222 |
| Vb.1 | H | Me | H | H | 3-(pyridin-4-yl) | 315-316 |
| Va.2 | H | F | H | H | 2-(4-fluorophényl) | 220-221 |
| Vb.2 | H | Cl | H | H | 3-(pyridin-4-yl) | 336 - 342 |
| Va.3 | H | Cl | H | H | 2-(pyridin-4-yl) | 190 - 216 |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans les tableaux 1 et 2. Les microanalyses élémentaires, les analyses LC-MS (chromatographie liquide couplée à la spectrométrie de masse) les spectres I.R. et R.M.N. confirment les structures des composés obtenus.

### Exemple 1 (Composé N°1)

### N,N-diéthylcarbamate de 7-fluoro-2-(4-fluorophényl)-4,5-dihydro-2H-benzo[e]indazol-1-yle

### 1.1 6-Fluoro-2-hydroxy-3,4-dihydro-naphtalén-1-yloate de méthyle

Dans un réacteur de 2 L sont introduits 12,66 g (316 mmoles) d'hydrure de sodium à 60% dans l'huile, 900 mL de toluène et 17,69 mL (210 mmoles) de diméthylcarbonate. Le mélange réactionnel est agité pendant 1 h à reflux. Une solution de 19 g (115 mmoles) de 6-fluoro-3,4-dihydro-1*H*-naphtalen-2-one dans 350 mL de toluène est ensuite ajoutée. Le mélange réactionnel est chauffé à reflux pendant 24h. Le mélange réactionnel est ensuite refroidi à 0°C puis acidifié par ajout de 114 mL d'acide acétique. 114 mL d'eau sont ajoutés et la phase organique séparée est décantée, lavée avec deux fois 150 mL d'eau puis avec 100 mL d'une solution aqueuse saturée en chlorure de sodium. La phase organique est ensuite séchée sur sulfate de magnésium puis concentrée sous pression réduite pour conduire à 26,1 g d'un produit qui est engagé tel quel dans l'étape suivante.

### 1.2 7-fluoro-2-(4-fluorophényl)-1-hydroxy-4,5-dihydro-2H-benzo[e]indazole (Va.2)

Dans un réacteur de 100 mL on introduit 2 g (9 mmoles) de produit obtenu à l'étape 1.1 et 2,73 g (16,8 mmoles) de chlorhydrate de 4-fluorophénylhydrazine. Le mélange est solubilisé dans 100 mL d'acide acétique et chauffé à reflux pendant 4h. Le mélange réactionnel est ensuite refroidi puis concentré sous pression réduite. Le résidu est repris dans 150 mL d'acétate d'éthyle et 100 mL d'eau. La phase organique est décantée, lavée deux fois avec 100 mL d'eau puis une fois avec 100 mL d'une solution aqueuse saturée en chlorure de sodium. La phase organique est ensuite séchée sur sulfate de magnésium puis concentrée sous pression réduite pour conduire à 3,5 g du composé attendu.
Point de fusion : 220 - 221 °C
R.M.N. ¹H (DMSO D6) : δ (ppm) : 2,72 (dxd, 2H), 2,95 (dxd, 2H), 7,01 (m, 2H), 7,3 (m, 2H), 7,75 (m, 3H).

### 1.3 N,N-diéthylcarbamate de 7-fluoro-2-(4-fluorophényl)-4,5-dihydro-2H-benzo[e]indazol-1-yle (Composé n°1)

On introduit, dans un réacteur de 500 mL, 3,5 g (9 mmoles) de produit obtenu à l'étape 1.2, 3,48 g (25 mmoles) de carbonate de potassium et 2,66 mL (21 mmoles) de chlorure de N,N-diéthylcarbamoyle. Le mélange réactionnel est chauffé pendant 24h à reflux puis concentré sous pression réduite. Le produit résultant est repris dans 100 mL d'acétate d'éthyle. La phase organique est lavée deux fois avec 100 mL d'eau puis une fois avec 100 mL d'une solution aqueuse saturée en chlorure de sodium. La phase organique est ensuite séchée sur sulfate de magnésium puis concentrée sous pression réduite pour conduire à 5,96 g de produit brut. Le mélange est purifié par chromatographie sur une colonne de silice en éluant avec un mélange de cyclohexane et d'acétate d'éthyle. 2,2 g du produit attendu sont ainsi isolés et recristallisés dans l'isopropanol pour obtenir 1,5 g (3,77 mmoles) de produit final.
Point de fusion : 141 -142 °C
R.M.N. ¹H (CDCl₃) : δ (ppm) : 1,95 (t, 3H), 2,6 (t, 3H), 2,97 (t, 2H), 3,98 (t, 2H), 3,4 (q, 2H), 3,52 (q, 2H), 7,00 (m, 2H), 7,2 (m, 2H), 7,32 (m , 1 H), 7,56 (m, 2H).

### Exemple 2 (Composé N°2)

### N,N-diéthylcarbamate de 7-fluoro-2-(4-fluorophényl)-2H-benzo[e]indazol-1-yle

Une solution de 0,7 g (1,76 mmoles) du N,N-diéthylcarbamate de 7-fluoro-2-(4-fluorophényl)-4,5-dihydro-2*H*-benzo[e]indazol-1-yle, obtenu à l'étape 1.3 de l'exemple 1, et de 1,2 g (5,2 mmoles) de 2,3-dichloro-5,6-dicyano-1,4-benzoquinone dans 25 mL de toluène est agitée pandant 2h à reflux puis refroidie. Le mélange est versé sur 100 mL d'acétate d'éthyle. Cette phase organique est lavée avec deux fois 100 mL d'une solution aqueuse saturée en hydrogénocarbonate de sodium, avec 100 mL d'eau puis 100 mL d'une solution aqueuse saturée en chlorure de sodium. La phase organique est séparée par décantation puis séchée sur sulfate de magnésium et concentrée sous pression réduite. Après purification par chromatographie sur colonne de silice (éluant : mélange de chlorure de méthylène et d'acétate d'éthyle) et recristallisation dans l'isopropanol, 500 mg (1,26 mmoles) du produit attendu sont obtenus.
Point de fusion : 159 - 160 °C
R.M.N. ¹H (DMSO) : δ (ppm) : 1,04 (t, 3H), 1,21 (t, 3H), 3,28 (q, 2H), 3,55 (q, 2H), 7,45 (m, 3H), 7,7 (m, 5H), 7,9 (dxd , 1 H).

### Exemple 3 (Composés n°3 et 4).

### Chlorhydrate de N,N-diéthylcarbamate de 7-chloro-2-(pyridin-4-yl)-4,5-dihydro-2H-benzo[e]indazol-1-yle (Composé n°3) et chlorhydrate de N,N-diéthylcarbamate de 7-chloro-3-(pyridin-4-yl)-4,5-dihydro-3H-benzo[e]indazol-1-yle (Composé n°4)

### 3.1 6-chloro-2-hydroxy-3,4-dihydro-naphtalén-1-yloate de méthyle

Dans un réacteur de 2 L sont introduits 10,1 g (252 mmoles) d'hydrure de sodium à 60% dans l'huile, 621 mL de toluène et 14,18 mL (163 mmoles) de diméthylcarbonate. Le mélange réactionnel est agité pendant 1h à reflux. Une solution de 15,2 g (84 mmoles) de 6-chloro-3,4-dihydro-1*H*-naphtalen-2-one dans 268 mL de toluène est ensuite ajoutée. Le mélange réactionnel est chauffé à reflux pendant 24h. Le mélange réactionnel est ensuite refroidi à 0°C puis acidifié par ajout de 92 mL d'acide acétique. 114 mL d'eau sont ajoutés et la phase organique séparée est décantée, lavée avec trois fois 150 mL d'eau puis avec 100 mL d'une solution aqueuse saturée en chlorure de sodium. La phase organique est ensuite séchée sur sulfate de magnésium puis concentrée sous pression réduite. Le résidu est purifié par chromatographie sur colonne de silice (en éluant avec un mélange de cyclohexane et de dichlorométhane) pour conduire à 12,8 g (53,6 mmoles) du produit attendu engagé tel quel dans l'étape suivantes

### 3.2 7-chloro-1-hydroxy-4,5-dihydro-2H-benzo[e]indazole

Dans un réacteur de 2 L on introduit 28 g (117 mmoles) de produit obtenu à l'étape 3.1 et 28,6 mL (586,6 mmoles) d'hydrazine monohydrate. Le mélange est solubilisé dans 782 mL d'acide acétique est chauffé à reflux durant 4h. Le mélange réactionnel est ensuite refroidi puis concentré sous pression réduite. Le produit résultant est repris dans 300 mL d'acétate d'éthyle et 300 mL d'eau. La phase organique est décantée, lavée deux fois avec 200 mL d'eau puis une fois avec 200 mL d'une solution aqueuse saturée en chlorure de sodium. La phase organique est ensuite séchée sur sulfate de magnésium puis concentrée sous pression réduite. Le résidu obtenu est trituré dans 200 mL d'éther éthylique puis filtré pour conduire à 25 g (113,3 mmoles) de produit attendu.
Point de fusion : 232 - 233 °C

### 3.3 7-chloro-1-hydroxy-2-(pyridin-4-yl)-4,5-dihydro-2H-benzo[e]indazole et 7-chloro-1-hydroxy-3-(pyridin-4-yl)-4,5-dihydro-3H-benzo[e]indazole.

Dans un réacteur de 2 L sont introduits, sous atmosphère inerte, 15,2 g (68,8 mmoles) de produit obtenu à l'étape 3.2, 16,95 g (82,66 mmoles) de 4-iodopyridine, 4,14 mL (34,44 mmoles) de trans-1,2-diaminocyclohexane, 1,31 g (6,89 mmoles) de iodure de cuivre et 36,55 g (172,2 mmoles) de phosphate de potassium. Le mélange réactionnel est mis en suspension dans 690 mL de dioxane, porté au reflux pendant 24h puis refroidi. Le mélange est concentré sous pression réduite puis repris dans 200 mL d'eau. Cette phase aqueuse est acidifiée à pH 5 par ajouts successifs d'acide acétique. La suspension est agitée pendant 30 minutes puis le précipité obtenu est filtré, lavé à l'eau puis séché sous pression réduite pour obtenir 16,6 g (55,7 mmoles) du produit de N-arylation attendu sous la forme d'un mélange d'isomères.
LC-MS : 2 pics à 60,4% et 38% correspondants à [MH]⁺= 298.

### 3.4 Chlorhydrate de N,N-diéthylcarbamate de 7-chloro-2-(pyridin-4-yl)-4,5-dihydro-2H-benzo[e]indazol-1-yle (Composé n°3) et chlorhydrate de N,N-diéthylcarbamate de 7-chloro-3-(pyridin-4-yl)-4,5-dihydro-3H-benzo[e]indazol-1-yle (Composé n°4)

Dans un réacteur de 2 L sont introduits, sous atmosphère inerte, 13 g (43,66 mmoles) du mélange d'isomères obtenu à l'étape 3.3, 18,1 g (131 mmoles) de carbonate de potassium finement broyé et 11,07 mL (87,32 mmoles) de chlorure de N,N-diéthylcarbamoyle. Le mélange réactionnel est mis en suspension dans 1 L d'acétonitrile, porté au reflux durant 24h puis refroidi. Le mélange réactionnel est concentré sous pression réduite. Le produit résultant est repris dans 300 mL d'acétate d'éthyle et 300 mL d'eau. La phase organique est décantée, lavée deux fois avec 200 mL d'eau puis une fois avec 200 mL d'une solution aqueuse saturée en chlorure de sodium. La phase organique est ensuite séchée sur sulfate de magnésium puis concentrée sous pression réduite. Le produit résultant contient les deux isomères de position (composés n°3 et 4). Ceux-ci sont séparés par chromatographie sur colonne (300g de silice Merck 15 - 40 microns, éluants : mélange d'heptane et d'acétate d'éthyle).

### Chlorhydrate de N,N-diéthylcarbamate de 7-chloro-2-(pyridin-4-yl)-4,5-dihydro-2H-benzo[e]indazol-1-yle (Composé n°3)

Le produit n°3, ainsi isolé, est transformé en chlorhydrate par solubilisation dans une solution d'acide chlorhydrique 0,1N dans l'isopropanol. La solution est concentrée à sec sous pression réduite. Après trituration dans l'éther éthylique, filtration et séchage sous pression réduite, 2,3 g (5,79 mmoles) de produit final sont obtenus..
Point de fusion : 250 - 251 °C
R.M.N. ¹H (DMSO) : δ (ppm) : 1,08 (t, 3H), 1,31 (t, 3H), 2.98 (m, 4H), 3,3 (q, 2H), 3,68 (q, 3H), 7,27 (d, 1 H), 7, 38 (dxd, 1 H), 7,46 (dxd, 1 H), 7,92 (d, 2H), 8,87 (d, 2H).

### Chlorhydrate de N,N-diéthylcarbamate de 7-chloro-3-(pyridin-4-yl)-4,5-dihydro-3H-benzo[e]indazol-1-yle (Composé n°4)

Le produit n°4, ainsi isolé, est transformé en chlorhydrate par solubilisation dans une solution d'acide chlorhydrique 0,1N dans l'isopropanol. La solution est concentrée à sec sous pression réduite. Après trituration dans l'éther éthylique, filtration et séchage sous - pression réduite, 6,2 g (15,62 mmoles) de produit final sont obtenus.
Point de fusion : 224 - 226°C
R.M.N. ¹H (DMSO) : δ (ppm) : 1,18 (t, 3H), 1,29 (t, 3H), 3,02 (t, 2H), 3,31 (m, 4H), 3,51 (q, 2H), 7,20 (d, 1H), 7,32 (dxd, 1H), 7,45 (d , 1H), 8,08 (d, 2H), 8,98 (d, 2H).

### Exemple 4 (Composé n°5)

### Chlorhydrate de N,N-diéthylcarbamate de 8-méthoxy-3-(pyridin-4-yl)-4,5-dihydro-3H-benzo[e]indazol-1-yle

### 4.1 6-méthoxy-2-hydroxy-3,4-dihydro-naphtalén-1-yloate de méthyle

Dans un réacteur de 1 L sont introduits 3,4 g (85,12 mmoles) d'hydrure de sodium à 60% dans l'huile, 180 mL de toluène et 4,78 mL (56,75 mmoles) de diméthylcarbonate. Le mélange réactionnel est agité pendant 1 h à reflux. Une solution de 5 g (28,37 mmoles) de 7-méthoxy-3,4-dihydro-1*H*-naphtalen-2-one dans 100 mL de toluène est ensuite ajoutée. Le mélange réactionnel est chauffé à reflux pendant 24h. Le mélange réactionnel est ensuite refroidi à 0°C puis acidifié par ajout de 30 mL d'acide acétique. 30 mL d'eau sont ajoutés et la phase organique séparée est décantée, lavée avec deux fois 50 mL d'eau puis avec 50 mL d'une solution aqueuse saturée en chlorure de sodium. La phase organique est ensuite séchée sur sulfate de magnésium puis concentrée sous pression réduite. Après purification par chromatographie sur colonne de silice (éluant : mélange de chlorure de méthylène et d'heptane) 3,9 g (16,64 mmoles) du produit attendu sont obtenus et engagés tel quel dans l'étape suivante.

### 4.2 1-hydroxy-8-méthoxy-4,5-dihydro-2H-benzo[e]indazole

Dans un réacteur de 0,5 L on introduit 3,9 g (16,65 mmoles) de produit obtenu à l'étape 4.1 et 4,06 mL (83,24 mmoles) d'hydrazine monohydrate. Le mélange est solubilisé dans 166 mL d'acide acétique et chauffé à reflux pendant 4h. Le mélange réactionnel est ensuite refroidi puis concentré sous pression réduite. Le produit résultant est repris dans 100 mL d'acétate d'éthyle et 100 mL d'eau. La phase organique est décantée, lavée deux fois avec 100 mL d'eau puis une fois avec 100 mL d'une solution aqueuse saturée en chlorure de sodium. La phase organique est ensuite séchée sur sulfate de magnésium puis concentrée sous pression réduite. Le résidu obtenu est trituré dans 50 mL d'éther éthylique puis filtré pour conduire à 2,4 g (11,1 mmoles) de produit attendu.

### 4.3 1-hydroxy-8-méthoxy-3-(pyridin-4-yl)-4,5-dihydro-3H-benzo[e]indazole

Dans un réacteur de 0,1 L sont introduits, sous atmosphère inerte, 1,2 g (5,55 mmoles) de produit obtenu à l'étape 4.2, 1,36 g (6,66 mmoles) de 4-iodopyridine, 0,33 mL (2,77 moles) de trans-1,2-diaminocyclohexane, 0,105 g (0,55 mmoles) de iodure de cuivre et 2,94 g (13,87 mmoles) de phosphate de potassium. Le mélange réactionnel est mis en suspension dans 55 mL de dioxane, porté au reflux pendant 24h puis refroidi. Le mélange est ensuite repris dans 1 L d'un mélange 1/1 d'eau et d'acétate d'éthyle. La phase organique est décantée puis lavée à l'eau (50 mL). Cette phase aqueuse est acidifiée à pH 5 par ajouts successifs d'acide acétique. Le précipité obtenu est filtré et lavé à l'eau puis séché sous pression réduite pour obtenir 0,2 g (0,68 mmoles) du produit de N-arylation attendu. La phase organique est séchée sur sulfate de magnésium puis concentrée sous pression réduite. Le produit résultant est purifié par chromatographie sur colonne de silice (éluants : mélange de dichlorométhane et méthanol) pour obtenir 0,57 g (1,94 mmoles) supplémentaires de produit de N-arylation attendu.
R.M.N. ¹H (DMSO D6) : δ (ppm) : 2,89 (dxd, 2H), 3,09 (dxd, 2H), 3,73 (s, 3H), 6,2 (dxd, 1H), 7,1 (m, 2H), 7,48 (m, 2H), 8,6 (m, 2H).

### 4.4 N,N-diéthylcarbamate de 8-méthoxy-3-(pyridin-4-yl)-4,5-dihydro-3H-benzo[e]indazol-1-yle (Composé n°5)

Dans un réacteur de 0,1 L sont introduits, sous atmosphère inerte, 0,77 g (2,63 mmoles) de produit obtenu à l'étape 4.3, 1,09 g (7,88 mmoles) de carbonate de potassium finement broyé et 0,67 mL (5,25 mmoles) de chlorure de N,N-diéthylcarbamoyle. Le mélange réactionnel est mis en suspension dans 30 mL d'acétonitrile, porté au reflux pendant 24h puis refroidi. Le mélange réactionnel est concentré sous pression réduite. Le produit résultant est repris dans 100 mL d'acétate d'éthyle et 100 mL d'eau. La phase organique est décantée, lavée deux fois avec 50 mL d'eau puis une fois avec 50 mL d'une solution aqueuse saturée en chlorure de sodium. La phase organique est ensuite séchée sur sulfate de magnésium puis concentrée sous pression réduite. Le produit résultant est purifié par chromatographie sur colonne (90g silice Merck 15 - 40 microns, éluants : mélange d'heptane et d'acétate d'éthyle). Le composé ainsi isolé, est recristallisé dans l'isopropanol puis resolubilisé dans une solution d'acide chlorhydrique 0,1N dans l'isopropanol. La solution est concentrée à sec sous pression réduite. Après trituration dans l'éther éthylique, filtration et séchage sous pression réduite, 234 mg (0.59 mmole) du composé attendu sont obtenus. Point de fusion : 225 - 227 °C .
R.M.N. ¹H (DMSO) : δ (ppm) : 1,15 (t, 3H), 1,3 (t, 3H), 2.92 (t, 2H), 3,2 - 3,4 (m, 4H), 3,51 (q, 2H), 3,71 (s, 3H), 6,8 (m, 2H), 7,2 (d , 1 H), 7,98 (d, 2H), 8,82 (d, 2H).

### Exemple 5 (Composé n°82)

### Chlorhydrate de N,N-diisopropylcarbamate de 7-chloro-4,5-dihydro-3-(pyridin-4-yl)-3H-benzo[e]indazol-1-yle

Dans un réacteur de 1 L, on additionne sous atmosphère inerte, goutte à goutte et à 0°C, sur une suspension de 0,4 g (10,04 mmoles) d'hydrure de sodium dans 17 mL de diméthylformamide, 2,3 g (7,72 mmoles) du mélange d'isomères obtenu à l'étape 3.3 de l'exemple 3, en solution dans 30 mL de diméthylformamide. Après 1 heure d'agitation à température ambiante sont additionnés, goutte à goutte, 1,39 g (8,5 mmoles) de chlorure de N,N-diisopropylcarbamoyle dans 30 mL de diméthylformamide. Le mélange réactionnel est agité pendant 18h à température ambiante puis est concentré sous pression réduite. Le produit résultant est repris dans 300 mL d'acétate d'éthyle et 300 mL d'eau. La phase aqueuse est amenée pH 5 par ajout d'acide acétique. La phase organique est décantée, lavée deux fois avec 200 mL d'eau puis une fois avec 200 mL d'une solution aqueuse saturée en chlorure de sodium. La phase organique est ensuite séchée sur sulfate de magnésium puis concentrée sous pression réduite. Le produit résultant est purifié par chromatographie sur colonne (300g de silice Merck 15 - 40 microns, éluants : mélange de dichlorométhane et d'acétate d'éthyle).
Le produit ainsi isolé, est transformé en chlorhydrate par solubilisation dans une solution d'acide chlorhydrique 0,1N dans l'isopropanol. La solution est concentrée à sec sous pression réduite. Après trituration dans l'éther éthylique, filtration et séchage sous pression réduite, 1,2 g (2,6 mmoles) de produit final sont obtenus.
Point de fusion : 237 - 269 °C
R.M.N. ¹H (DMSO) : δ (ppm) : 1,29 (m, 12H), 3.02 (m, 2H), 3,19 (m, 2H), 3.91 (m, 1H), 4,2 (m, 1H), 7,2 (d, 1 H),7,35 (dxd, 1H), 7,42 (s, 1H), 7, 6 (d, 2H), 8,7 (d, 2H).

### Exemple 6 (Composé n°84)

### Chlorhydrate de N,N-diisopropylcarbamate de 7-chloro-3-(pyridin-4-yl)-3H-benzo[e]indazol-1-yle

Dans un réacteur de 100 mL, on additionne 1,65 g (3,38 mmoles) de N,N-diisopropylcarbamate de 7-chloro-4,5-dihydro-3-(pyridin-4-yl)-3*H*-benzo[e]indazol-1-yle, préparé selon la méthode décrite à l'exemple 5, 1,105 g (6,21 mmoles) de N-bromosuccinimide et 0,127 g (0,78 mmole) de 2,2'-azobis(2-methylpropionitrile). Le mélange, mis en solution dans 40 mL de tétrachlorure de carbone, est agité pendant 24h au reflux puis concentré sous pression réduite. Le produit résultant est repris dans 300 mL de dichlorométhane et 2 mL d'une solution aqueuse d'ammoniaque concentrée. La phase organique est séparée, lavée avec 200 mL d'eau puis avec 200 mL d'une solution aqueuse saturée en chlorure de sodium. La phase organique est ensuite séchée sur sulfate de magnésium puis concentrée sous pression réduite. Le produit résultant est purifié par chromatographie sur colonne d'alumine (éluants: mélange de dichlorométhane et méthanol) puis sur une colonne de silice (éluants : mélange de dichlorométhane et d'acétate d'éthyle).
Le produit ainsi isolé, est transformé en chlorhydrate par solubilisation dans une solution d'acide chlorhydrique 0,1N dans l'isopropanol. La solution est concentrée à sec sous pression réduite. Après trituration dans l'éther éthylique, filtration et séchage sous pression réduite, 1,2 g (2,6 mmoles) de produit final sont obtenus.
Point de fusion : 234 - 248 °C
R.M.N. ¹H (DMSO) : δ (ppm) : 1,42 (m, 12H), 4.02 (m, 1H), 4.41 (m, 1H), 7,6 (dxd, 1H), 7,8 (m, 3H), 7,92 (d, 2H), 8,15 (d, 1H), 8,79 (d, 2H).

Le tableau 2 qui suit illustre les structures chimiques et les propriétés physiques de quelques composés de formule générale (I) selon l'invention.
Dans la colonne "Sel" de ce tableau, "HCl" désigne un chlorhydrate, "-" désigne un composé à l'état de base. Les rapports molaires acide : base sont indiqués en regard. La colonne "PF" renseigne les points de fusion des produits, les composés amorphes étant caractérisés par leurs résultats d'analyse par spectrométrie de masse (MS).

**Tableau 2**

| | | | | | | | | | | |
|---|---|---|---|---|---|---|---|---|---|---|
| 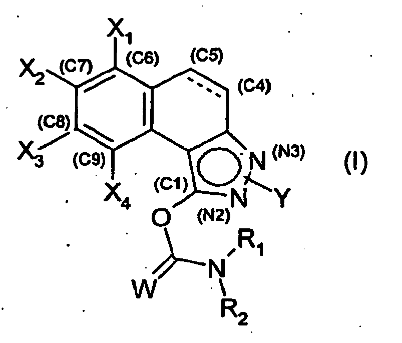 | | | | | | | | | | |

| **n°** | **X₁** | **X₂** | **X**₃ | **X₄** | **w** | **NR₁R₂** | **Liaison C4-C5** | **Y** | **Sel** | **PF (°C)** |
|---|---|---|---|---|---|---|---|---|---|---|
| 1 | H | F | H | H | O | N(CH₂CH₃)₂ | simple | 2-(4-fluorophényl) | - | 141-142 |
| 2 | H | F | H | H | O | N(CH₂CH₃)₂ | double | 2-(4-fluorophényl) | - | 159-160 |
| 3 | H | Cl | H | H | O | N(CH₂CH₃)₂ | simple | 2-(pyridin-4-yl) | HCl 1:1 | 250-251 |
| 4 | H | Cl | H | H | O | N(CH₂CH₃)₂ | simple | 3-(pyridin-4-yl) | HCl 1:1 | 224-226 |
| 5 | H | H | OCH₃ | H | O | N(CH₂CH₃)₂ | simple | 3-(pyridin-4-yl) | HCl 1:1 | 225-227 |
| 6 | H | H | H | H | O | N(CH₃)₂ | simple | 2-(4-méthylphényl) | - | 130-131 |
| 7 | H | H | H | H | O | N(CH₂CH₃)₂ | simple | 2-(4-chlorophényl) | - | 148-149 |
| 8 | H | H | H | H | O | 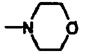 | simple | 2-(3-chlorophényl) | - | 150-151 |
| 9 | H | H | H | H | O | N(CH₃)₂ | simple | 2-(3-chlorophényl) | - | 128-129 |
| 10 | H | H | H | H | O | N(CH₂CH₃)₂ | simple | 2-(3-chlorophényl) | - | 111-113 |
| 11 | H | H | H | H | O | 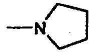 | simple | 2-(3-chlorophényl) | - | 155-157 |
| 12 | H | H | H | H | O | N(CH(CH₃)₂)₂ | simple | 2-(3-chlorophényl) | - | 116-118 |
| 13 | H | H | H | H | O | N(CH₂CH₃)₂ | simple | 2-phényl | - | 124-125 |
| 14 | H | Cl | H | H | O | N(CH₂CH₃)₂ | simple | 2-phényl | - | 114-116 |
| 15 | H | H | H | H | O | N(CH₃)Ph | simple | 2-(3-chlorophényl) | - | 75-78 |
| 16 | H | H | H | H | O | N(CH₂CH₃)₂ | simple | 2-(2-chlorophényl) | - | 396* |
| 17 | H | H | H | H | O | N(CH₂CH₃)₂ | simple | 2-éthyl | - | 60-61 |
| 18 | H | H | H | H | O | N(CH₃)Ph | simple | 2-éthyl | - | 348* |
| 19 | H | H | H | H | O | N(CH₃)Ph | simple | 2-(2,2',2"-trifluoroéthyl) | - | 402* |
| 20 | H | H | H | H | O | N(CH₂CH₃)₂ | simple | 2-(4-fluorophényl) | - | 132-133 |
| 21 | H | H | H | H | O | N(CH(CH₃)₂)₂ | simple | 2-(4-fluorophényl) | - | 95-96 |
| 22 | H | H | H | H | O | N(CH₃)Ph | simple | 2-(4-fluorophényl) | - | 197-198 |
| 23 | H | H | H | H | O | 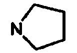 | simple | 2-(4-fluorophényl) | - | 211-212 |
| 24 | H | H | H | H | O | 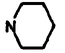 | simple | 2-(4-fluorophényl) | - | 174-175 |
| 25 | H | H | H | H | O | 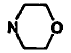 | simple | 2-(4-fluorophényl) | - | 175-176 |
| 26 | H | H | H | H | O | N(CH₃)₂ | simple | 2-(4-chlorophényl) | - | 211-212 |
| 27 | H | H | H | H | O | N(CH(CH₃)₂)₂ | simple | 2-(4-chlorophényl) | - | 203-204 |
| 28 | H | H | H | H | O | N(CH₃)Ph | simple | 2-(4-chlorophényl) | - | 202-203 |
| 29 | H | H | H | H | O | 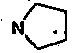 | simple | 2-(4-chlorophényl) | - | 212-213 |
| 30 | H | H | H | H | O | 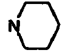 | simple | 2-(4-chlorophényl) | - | 200-201 |
| 31 | H | H | H | H | O | 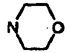 | simple | 2-(4-chlorophényl) | - | 185-186 |
| 32 | H | H | H | H | O | 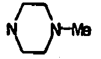 | simple | 2-(4-chlorophényl) | - | 157-158 |
| 33 | H | H | H | | O | N(CH₃)₂ | simple | 2-(4-fluorophényl) | - | 216-217 |
| 34 | H | H | H | H | O | 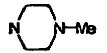 | simple | 2-(4-fluorophényl) | - | 167-168 |
| 35 | H | H | H | H | O | N(CH₃)₂ | simple | 2-phényl | - | 158-159 |
| 36 | H | H | H | H | O | N(CH(CH₃)₂)₂ | simple | 2-phényl | - | 146-147 |
| 37 | H | H | H | H | O | N(CH₃)Ph | simple | 2-phényl | - | 169-170 |
| 38 | H | H | H | H | O | 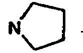 | simple | 2-phényl | - | 176-177 |
| 39 | H | Cl | H | H | O | N(CH₂CH₃)₂ | simple | 2-(4-fluorophényl) | - | 155-156 |
| 40 | H | H | H | H | O | N(CH₃) (CH₂)₂CH₃ | simple | 2-(4-fluorophényl) | - | 143-144 |
| 41 | H | H | H | H | O | N(CH₂CH₃)₂ | simple | 2-(3-chloro-4-méthylphényl) | - | 99-101 |
| 42 | H | H | H | H | O | N(CH₂CH₃)₂ | simple | 2-(3-chloro-4-fluorophényl) | - | 148-150 |
| 43 | H | Cl | H | H | O | N(CH₂CH₃)₂ | simple | 2-(3-chloro-4-méthylphényl) | - | 131-133 |
| 44 | H | Cl | H | H | O | N(CH₂CH₃)₂ | simple | 2-(2,4-difluorophényl) | - | 78-80 |
| 45 | H | H | H | H | O | N(CH₂CH₃)₂ | simple | 2-(pyridin-4-yl) | HCl 1 :1 | 298-299 |
| 46 | H | Cl | H | H | O | N(CH₂CH₃)₂ | simple | 2-(pyridin-2-yl) | - | 108-110 |
| 47 | H | OCH₃ | H | H | O | N(CH₂CH₃)₂ | simple | 2-(4-fluorophényl) | - | 161-162 |
| 48 | H | Br | H | H | O | N(CH₂CH₃)₂ | simple | 2-(4-fluorophényl) | - | 162-163 |
| 49 | H | Cl | H | H | O | N(CH₂CH₃)₂ | simple | 2-(3-chloro-4-fluorophényl) | - | 108-110 |
| 50 | H | Cl | H | H | O | N(CH₂CH₃)₂ | simple | 2-méthyl | - | 117-118 |
| 51 | H | H | H | H | O | N(CH₂CH₃)₂ | double | 2-(3-chlorophényl) | - | 125-126 |
| 52 | H | H | H | H | O | N(CH₂CH₃)₂ | double | 2-(4-fluorophényl) | - | 125-126 |
| 53 | H | H | H | H | O | N(CH₃)(CH₂)₂ CH₃ | double | 2-(4-fluorophényl) | - | 154-155. |
| 54 | H | H | H | H | O | N(CH₂CH₃)₂ | double | 2-(3-chloro-4-fluorophényl) | - | 412* |
| 55 | H | H | H | H | O | N(CH₂CH₃)₂ | double | 2-(3-chloro-4-méthylphényl) | - | 118-120 |
| 56 | H | Cl | H | H | O | N(CH₂CH₃)₂ | double | 2-(4-fluorophényl) | - | 151-153 |
| 57 | H | Cl | H | H | O | N(CH₂CH₃)₂ | double | 2-(3-chloro-4-fluorophényl) | - | 141-143 |
| 58 | H | Cl | H | H | O | N(CH₂CH₃)₂ | double | 2-(3-chloro-4-méthylphényl) | - | 141-143 |
| 59 | H | Cl | H | H | O | N(CH₂CH₃)₂ | double | 2-(2,4-difluorophényl) | - | 144-146 |
| 60 | H | OCH₃ | H | H | O | N(CH₂CH₃)₂ | simple | 3-(pyridin-4-yl) | HCl 1 :1 | 208-220 |
| 61 | H | CH₃ | H | H | O | N(CH₂CH₃)₂ | simple | 3-(pyridin-4-yl) | HCl 1 :1 | 222-225 |
| 62 | H | Cl | H | H | O | N(CH₂CH₃)₂ | simple | 3-(pyridin-3-yl) | HCl 1 :1 | 194-197 |
| 63 | H | Cl | H | H | O | N(CH₂CH₃)₂ | simple | 2-(pyridin-3-yl) | HCl 1:1 | 155-157 |
| 64 | H | F | H | H | O | N(CH₂CH₃)₂ | simple | 2-(pyridin-4-yl) | HCl 1 :1 | 324-326 |
| 65 | H | F | H | H | O | N(CH₂CH₃)₂ | simple | 3-(pyridin-4-yl) | HCl 1 :1 | 233-242 |
| 66 | H | Cl | H | H | O | N(CH₂CH₃)₂ | simple | 3-(4-fluorophényl) | - | 131-132 |
| 67 | H | Cl | H | H | O | N(CH₂CH₃)₂ | simple | 3-(pyridin-2-yl) | HCl 1:1 | 122-123 |
| 68 | H | Cl | H | H | O | N(CH₂CH₃)₂ | simple | 2-(3-fluorophényl) | - | 128-130 |
| 69 | H | Cl | H | H | O | N(CH₂CH₃)₂ | simple | 3-(3-fluorophényl) | - | 124-126 |
| 70 | H | Br | H | H | O | N(CH₂CH₃)₂ | simple | 2-(pyridin-4-yl) | HCl 1 :1 | 300-310 |
| 71 | H | Br | H | H | O | N(CH₂CH₃)₂ | simple | 3-(pyridin-4-yl) | HCl 1:1 | 233-238 |
| 72 | H | Cl | H | H | O | N(CH₂CH₃)₂ | simple | 3-(4-méthylphényl) | - | 155-159 |
| 73 | H | Cl | H | H | O | N(CH₂CH₃)₂ | simple | 2-(4-méthylphényl) | - | 118 -121 |
| 74 | H | Cl | H | H | O | N(CH₂CH₃)₂ | simple | 2-(2-fluorophényl) | - | 414* |
| 75 | H | Cl | H | H | O | N(CH₂CH₃)₂ | simple | 3-(4-chlorophényl) | - | 177 - 178 |
| 76 | H | Cl | H | H | O | N(CH₂CH₃)₂ | simple | 2-(4-chlorophényl) | - | 148 - 149 |
| 77 | H | Cl | H | H | O | N(CH₂CH₃)₂ | simple | 2-(4-méthoxyphényl) | - | 131-132 |
| 78 | H | Cl | H | H | O | N(CH₂CH₃)₂ | simple | 3-(4-méthoxyphényl) | - | 185-187 |
| 79 | H | Cl | H | H | O | N(CH₂CH₃)₂ | double | 3-(3-fluorophényl) | - | 142 - 144 |
| 80 | H | Cl | H | H | O | N(CH₃)Ph | simple | 3-(pyridin-4-yl) | - | 213 - 215 |
| 81 | H | Cl | H | H | O | N(CH(CH₃)₂)₂ | simple | 2-(pyridin-4-yl) | - | 184 - 185 |
| 82 | H | Cl | H | H | O | N(CH(CH₃)₂)₂ | simple | 3-(pyridin-4-yl) | - | 146 148 |
| | | | | | | | | | HCl 1 :1 | 237 - 269 |
| 83 | OC H₃ | H | H | H | O | N(CH₂CH₃)₂ | simple | 3-(pyridin-4-yl) | HCl 1:1 | 238 -240 |
| 84 | H | Cl | H | H | O | N(CH(CH₃)₂)₂ | double | 3-(pyridin-4-yl) | HCl 1:1 | 234-248 |
| 85 | H | Cl | H | H | O | 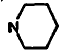 | simple | 2-(pyridin-4-yl) | HCl 1:1 | 153-173 |
| 86 | H | Cl | H | H | O | 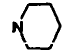 | simple | 3-(pyridin-4-yl) | HCl 1:1 | 243-248 |
| 87 | H | Cl | H | H | O | N(CH(CH₃)₂)₂ | simple | 2-(pyridin-3-yl) | HCl 1:1 | 243-260 |
| 88 | H | Cl | H | H | O | N(CH(CH₃)₂)₂ | simple | 3-(pyridin-3-yl) | HCl 1:1 | 209-213 |
| 89 | H | Cl | H | H | S | N(CH(CH₃)₂)₂ | simple | 3-(pyridin-4-yl) | HCl 1:1 | 290-318 |
| 90 | H | Cl | H | H | O | 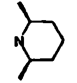 | simple | 3-(pyridin-4-yl) | HCl 1:1 | 228-252 |
| 91 | H | Cl | H | H | O | NCH₃C(CH₃) 3 | simple | 2-(pyridin-4-yl) | HCl 1:1 | 350-360 |
| 92 | H | Cl | H | H | O | N(CH(CH₃)₂)₂ | double | 3-(pyridin-3-yl) | HCl 1:1 | 238-240 |
| 93 | H | Cl | H | H | O | N(CH(CH₃)₂)₂ | simple | 3-(pyridin-2-yl) | - | 193-195 |
| 94 | H | F | H | H | O | N(CH(CH₃)₂)₂ | simple | 3-(pyridin-4-yl) | HCl 1:1 | 255-260 |
| 95 | H | Cl | H | H | O | N(CH(CH₃)₂)₂ | simple | 3-(pyrimidin-2-yl) | - | 220-222 |
| 96 | H | Cl | H | H | O | N(CH(CH₃)₂)₂ | simple | 2-(pyrazin-2-yl) | - | 175-176 |
| 97 | H | F | H | H | O | N(CH(CH₃)₂)₂ | double | 3-(pyridin-4-yl) | HCl 1:1 | 272-280 |
| 98 | H | Cl | H | H | O | N(CH(CH₃)₂)₂ | double | 3-(pyridin-2-yl) | - | 213-215 |
| 99 | H | Cl | H | H | O | N(CH₃) (C(CH₃)₃) | simple | 3-(pyridin-4-yl) | HCl 1:1 | 411* |
| 100 | H | Cl | H | H | O | N(CH₂CH₃)₂ | double | 3-(pyridin-4-yl) | HCl 1:1 | 227-229 |
| 101 | H | Cl | H | H | O | N(CH₃)Ph | double | 3-(pyridin-4-yl) | HCl 1:1 | 248-268 |

| | | | | | | | | | | |
|---|---|---|---|---|---|---|---|---|---|---|
| *[MH]⁺ | | | | | | | | | | |

Les composés de l'invention ont été soumis à des essais pharmacologiques qui ont mis en évidence leur intérêt comme substances à activités thérapeutiques.

Les composés de l'invention présentent également des caractéristiques de solubilité dans l'eau qui favorisent une bonne activité in vivo.

### Etude de la liaison [3H]Ro5-4864 aux récepteurs de type périphérique aux benzodiazépines (sites p ou PBR)

L'affinité des composés de l'invention pour les sites p ou PBR (sites de liaison de type périphérique aux benzodiazépines) a été déterminée.
Les récepteurs sites p peuvent être marqués sélectivement dans des membranes de rein de rat incubées en présence de [3H]Ro5-4864. Les composés de l'invention ont fait l'objet d'une étude in vitro quant à leur affinité pour ces récepteurs.
Les animaux utilisés sont des rats mâles Sprague Dawley (Iffa Credo) de 180 à 300 mg. Après décapitation, le rein est prélevé et le tissu est homogénéisé à 4°C au moyen d'un homogénéiseur PolytronTM pendant 2 min à 6/10 de la vitesse maximale, dans 35 volumes de tampon phosphate Na₂HPO₄ 50 mM, à un pH ajusté à 7,5 avec du NaH₂PO₄. L'homogénat membranaire est filtré sur gaze et dilué 10 fois avec du tampon.
Le [3H]Ro5-4864 (Activité spécifique : 70-90 Ci/mmol ; New England Nuclear), à une concentration de 0,5 nM, est incubé en présence de 100 µl de l'homogénat membranaire dans un volume final de 1 ml de tampon contenant le composé à tester. Après une incubation de 3 h à 0°C, les membranes sont récupérées par filtration sur filtres Whatman GF/BTM lavés avec 2 fois 4,5 ml de tampon d'incubation froid (0°C). La quantité de radioactivité retenue par le filtre est mesurée par scintigraphie liquide.
Pour chaque concentration de composé étudié, le pourcentage d'inhibition de la liaison du [3H]Ro5-4864, puis la concentration CI50, concentration qui inhibe 50% de la liaison spécifique, sont déterminés.
Les CI50 des composés les plus actifs de l'invention présentent des valeurs allant de 0,5 nM à 300 nM. Notamment les composés n° 14, 20 et 56 du tableau 2 présentent des CI50 respectives de 1,6 nM ;.2,8 nM et 1,4 nM.

Les composés de l'invention sont donc des ligands affins pour les récepteurs de type périphérique aux benzodiazepines.

### Etude de l'activité neuroprotectrice

### Test de survie des motoneurones après section du nerf facial chez le rat âgé de 4 jours

Après lésion du nerf facial chez le rat immature, les motoneurones du noyau facial subissent une mort neuronale par apoptose. L'évaluation de la survie neuronale est réalisée à l'aide de méthodes histologiques et comptage neuronal.
Des rats immatures de 4 jours sont anesthésiés au pentobarbital (3 mg/kg par voie i.p.). Le nerf facial droit est dégagé et sectionné, à sa sortie du foramen stylomastoïdien. Après le réveil, les ratons sont remis avec leur mère et traités, pendant 7 jours, par une ou deux administrations quotidiennes, par voie orale ou intrapéritonéale, à des doses allant de 1 à 10 mg/kg.
7 jours après la lésion, les animaux sont décapités, et les cerveaux congelés dans l'isopentane à -40°C. Le noyau facial est coupé au cryostat, en sections de 10 µm, dans sa totalité. Les motoneurones sont colorés au crésyl violet et comptés à l'aide du logiciel HistoTM (BiocomTM).
Dans ce modèle, les composés de l'invention augmentent la survie neuronale de 38 à 78 %. Le tableau 3 ci-dessous présente les résultats du test de survie des motoneurones pour les composés n° 14, 20 et 56 du tableau.

**Tableau 3**

| n° | 14 | 20 | 56 |
|---|---|---|---|
| % augmentation de la survie des neurones (10mg/kg po) | 38% | 59% | 74% |

Les résultats des essais montrent que les composés les plus actifs de l'invention favorisent la neuroprotection.

Les composés de l'invention peuvent donc être utilisés pour la préparation de médicaments, notamment pour la préparation d'un médicament destiné à prévenir ou à traiter une pathologie dans laquelle les récepteurs de type périphérique aux benzodiazépines sont impliqués.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvate.

Ces médicaments trouvent leur emploi en thérapeutique, notamment dans la prévention et/ou le traitement des neuropathies périphériques de différents types, comme les neuropathies traumatiques ou ischémiques, neuropathies infectieuses, alcooliques, diabétiques, médicamenteuses ou génétiques, ainsi que des affections du motoneurone, telle que les amyotrophies spinales et la sclérose latérale amyotrophique. Ces médicaments trouveront également une application dans le traitement des maladies neurodégénératives du système nerveux central, soit de type aigu comme les accidents vasculaires cérébraux et les traumatismes crâniens et médullaires, soit de type chronique comme les maladies auto-immunes (sclérose en plaques), la maladie d'Alzheimer, la maladie de Parkinson et toute autre maladie dans laquelle l'administration de facteurs neuroprotecteurs/neurotrophes est censée avoir un effet thérapeutique.

Les composés de l'invention peuvent également être utilisés pour la préparation de médicaments destinés à la prévention et/ou au traitement de l'anxiété, de l'épilepsie et des troubles du sommeil. En effet, les ligands des sites p ou PBR stimulent la production de neurostéroïdes tels que la pregnénolone, la déhydroepiandrostérone, et la 3-alphahydroxy-5-alphaprégnan-20-one en favorisant le transfert du cholestérol de l'extérieur à l'intérieur de la membrane mitochondriale. Ces neurostéroïdes modulent l'activité du complexe macromoléculaire GABA_{A}-canal chlorure et ainsi peuvent produire des activités anxiolytiques, anti-convulsivantes et sédatives.

Les composés de l'invention peuvent aussi être utilisés dans les traitements de l'insuffisance rénale aiguë ou chronique, de la glomérulonéphrite, de la néphropathie diabétique, de l'ischémie et de l'insuffisance cardiaques, de l'infarctus du myocarde, de l'ischémie des membres inférieurs, du vasospasme coronaire, de l'angine de poitrine, des pathologies associées aux valves cardiaques, des maladies cardiaques inflammatoires, des effets secondaires dus à des médicaments cardiotoxiques ou aux suites d'une chirurgie cardiaque, de l'athérosclérose et de ses complications thrombo-emboliques, de la resténose, des rejets de greffes, des conditions liées à une prolifération ou une migration incorrecte des cellules musculaires lisses.

Par ailleurs, des données récentes de la littérature indiquent que le récepteur de type périphérique aux benzodiazépines pourrait jouer un rôle fondamental dans la régulation de la prolifération cellulaire et les processus de cancérisation. D'une manière générale, et par comparaison avec des tissus normaux, une densité accrue de récepteurs de type périphérique aux benzodiazépines est observée dans différents types de tumeurs et cancers.

Dans les astocytomes humains, le niveau d'expression du récepteur de type périphérique aux benzodiazépines est corrélé avec le degré de malignité de la tumeur, l'index de prolifération et la survie des patients. Dans les tumeurs cérébrales humaines, l'augmentation du nombre de récepteurs de type périphérique aux benzodiazépines est utilisée comme une indication diagnostique en imagerie médicale et comme cible thérapeutique pour des conjugués formés d'un ligand du récepteur de type périphériques aux benzodiazépines et d'une drogue cytostatique. Une densité élevée de récepteurs de type périphérique aux benzodiazépines est également observée dans les carcinomes ovariens et les cancers du sein. Concernant ces derniers, il a été démontré que le niveau d'expression des récepteurs de type périphérique aux benzodiazépines est relié au potentiel agressif de la tumeur ; de plus la présence d'un agoniste du récepteur de type périphérique aux benzodiazépines stimule la croissance d'une lignée de cancer mammaire.

L'ensemble de ces résultats, qui suggère une fonction délétère du récepteur de type périphérique aux benzodiazépines dans les processus de cancérisation, constitue une base pertinente pour la recherche de ligands synthétiques spécifiques du récepteur de type périphérique aux benzodiazépines capables d'en bloquer les effets.

Les composés peuvent donc être utilisés pour le traitement des tumeurs et cancers.

Les récepteurs de type périphérique aux benzodiazépines sont également présents au niveau de la peau et, à ce titre, les composés utilisables selon l'invention peuvent être utilisés pour la prophylaxie ou le traitement des stress cutanés.
Par stress cutané, on entend les différentes situations qui pourraient provoquer des dommages en particulier au niveau de l'épiderme, quel que soit l'agent qui provoque ce stress. Cet agent peut être interne et/ou externe à l'organisme, comme un agent chimique ou radicalaire, ou bien externe, comme un rayonnement ultraviolet.
Ainsi les composés utilisables selon l'invention sont destinés à prévenir et à lutter contre les irritations cutanées, les dartres, les érythèmes, les sensations dysesthésiques, les sensations d'échauffement, les prurits de la peau et/ou des muqueuses, le vieillissement et peuvent aussi être utilisés dans les désordres cutanés tels que, par exemple, le psoriasis, les maladies prurigineuses, l'herpès, les photodermatoses, les dermatites atopiques, les dermatites de contact, les lichens, les prurigos, les prurits, les piqûres d'insectes, dans les fibroses et autres troubles de la maturation des collagènes, dans les désordres immunologiques ou encore dans des affections dermatologiques comme l'eczéma.

Les composés de l'invention peuvent également être utilisés pour la prévention et le traitement des maladies inflammatoires chroniques, notamment la polyarthrite rhumatoïde et les maladies inflammatoires pulmonaires.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par vole orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient

## Revendications

1. Composé répondant à la formule (I) 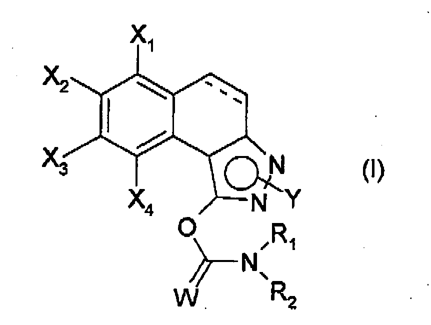 dans laquelle
W représente un atome d'oxygène ou de soufre ;
X₁, X₂, X₃ et X₄ représentent chacun, indépendamment l'un de l'autre, un atome d'hydrogène ou d'halogène ou un groupe cyano, C₁-C₆-alkyle, C₁-C₆-fluoroalkyle, C₁-C₆-alcoxy, C₁-C₆-fluoroalcoxy ;
Y est en position (N2) ou (N3) ;
quand Y est en position (N2), Y représente un groupe C₁-C₆-alkyle, C₁-C₆-fluoroalkyle, aryle comprenant entre 6 et 10 atomes de carbone ou hétéroaryle comprenant de 5 à 6 chaînons et comprenant entre 1 et 2 hétéroatomes;
quand Y est en position (N3), Y représente un groupe aryle ou hétéroaryle ;
les groupes aryle ou hétéroaryle étant éventuellement substitués par un ou plusieurs atomes ou groupes choisis parmi les atomes d'halogène, les groupes C₁-C₆-alkyle, C₁-C₆-alcoxy, C₁-C₆-alkylthio, C₁-C₆-alkyl-S(O)-, C₁-C₆-alkyl-S(O)₂-, C₁-C₆-fluoroalkyle ;
la liaison en position C4-C5 est double ou simple ;
R₁ et R₂ représentent chacun, indépendamment l'un de l'autre, un groupe aryle comprenant entre 6 et 10 atomes de carbone, benzyle ou C₁-C₆-alkyle ; ou bien R₁ et R₂ forment, avec l'atome d'azote qui les porte un hétérocycle comprenant de 4 à 7 chaînons et comprenant un atome d'azote et éventuellement un autre hétéroatome, éventuellement substitué par un ou plusieurs groupes C₁-C₆-alkyle ou benzyle ;
à l'état de base ou de sel d'addition à un acide, ainsi qu'à l'état d'hydrate ou de solvate.

2. Composé de formule (I) selon la revendication 1, **caractérisé en ce que**
W représente un atome d'oxygène ou de soufre ;
X₁, X₂ et X₃ représentent chacun, indépendamment l'un de l'autre, un atome d'hydrogène ou d'halogène ou un groupe cyano, C₁-C₆-alkyle, C₁-C₆-alcoxy ;
X₄ représente un atome d'hydrogène ;
Y est en position (N2) ou (N3) ;
quand Y est en position (N2), Y représente un groupe C₁-C₆-alkyle, C₁-C₆-fluoroalkyle, aryle comprenant entre 6 et 10 atomes de carbone ou hétéroaryle comprenant de 5 à 6 chaînons et comprenant entre 1 et 2 hétéroatomes;
quand Y est en position (N3), Y représente un groupe aryle ou hétéroaryle ;
les groupes aryle ou hétéroaryle étant éventuellement substitués par un ou plusieurs atomes ou groupes choisis parmi les atomes d'halogène, les groupes C₁-C₆-alkyle et C₁-C₆-alcoxy ;
la liaison en position C4-C5 est double ou simple ;
R₁ et R₂ représentent chacun, indépendamment l'un de l'autre, un groupe aryle comprenant entre 6 et 10 atomes de carbone, ou C₁-C₆-alkyle ; ou bien R₁ et R₂ forment, avec l'atome d'azote qui les porte, un hétérocycle, comprenant de 4 à 7 chaînons et comprenant un atome d'azote et éventuellement un autre hétéroatome, éventuellement substitué par un ou deux groupes C₁-C₆-alkyle ;
à l'état de base ou de sel d'addition à un acide, ainsi qu'à l'état d'hydrate ou de solvate.

3. Composé de formule (I) selon la revendication 1 ou 2, **caractérisé en ce que**
W représente un atome d'oxygène ou de soufre ;
X₁, X₂ et X₃ représentent chacun, indépendamment l'un de l'autre, un atome d'hydrogène ou d'halogène ou un groupe C₁-C₆-alkyle, C₁-C₆-alcoxy ;
X₄ représente un atome d'hydrogène ;
Y est en position (N2) ou (N3) et représente un groupe aryle comprenant entre 6 et 10 atomes de carbone ou hétéroaryle comprenant de 5 à 6 chaînons et comprenant entre 1 et 2 hétéroatomes;
les groupes aryle ou hétéroaryle étant éventuellement substitués par un ou plusieurs atomes ou groupes choisis parmi les atomes d'halogène, les groupes C₁-C₆-alkyle et C₁-C₆-alcoxy ;
la liaison en position C4-C5 est double ou simple ;
R₁ et R₂ représentent chacun, indépendamment l'un de l'autre, un groupe aryle comprenant entre 6 et 10 atomes de carbone, ou C₁-C₆-alkyle; ou bien R₁ et R₂ forment, avec l'atome d'azote qui les porte, un hétérocycle, comprenant de 4 à 7 chaînons et comprenant un atome d'azote et éventuellement un autre hétéroatome, éventuellement substitué par un ou deux groupes C₁-C₆-alkyle ;
à l'état de base ou de sel d'addition à un acide, ainsi qu'à l'état d'hydrate ou de solvate.

4. Composé de formule (I) selon l'une quelconque des revendications 1 à 3, **caractérisé en ce que**
W représente un atome d'oxygène ou de soufre ;
X₁, X₂ et X₃ représentent chacun, indépendamment l'un de l'autre, un atome d'hydrogène ou d'halogène ou un groupe C₁-C₆-alkyle, C₁-C₆-alcoxy ;
X₄ représente un atome d'hydrogène ;
Y est en position (N3) et représente un groupe aryle comprenant entre 6 et 10 atomes de carbone ou hétéroaryle comprenant de 5 à 6 chaînons et comprenant entre 1 et 2 hétéroatomes;
les groupes aryle ou hétéroaryle étant éventuellement substitués par un ou plusieurs atomes ou groupes choisis parmi les atomes d'halogène, les groupes C₁-C₆-alkyle et C₁-C₆-alcoxy ; la liaison en position C4-C5 est double ou simple ;
R₁ et R₂ représentent chacun, indépendamment l'un de l'autre, un groupe aryle comprenant entre 6 et 10 atomes de carbone, C₁-C₆-alkyle ; ou bien R₁ et R₂ forment, avec l'atome d'azote qui les porte, un hétérocycle, comprenant de 4 à 7 chaînons et comprenant un atome d'azote et éventuellement un autre hétéroatome, éventuellement substitué par un ou deux groupes C₁-C₆-alkyle ;
à l'état de base ou de sel d'addition à un acide, ainsi qu'à l'état d'hydrate ou de solvate.

5. Composé de formule (I) selon l'une quelconque des revendications 1 à 4, **caractérisé en ce qu'**il est choisi parmi :
*N,N*-Diéthylcarbamate de 7-fluoro-2-(4-fluorophényl)-4,5-dihydro-2*H*-benzo[*e*]indazol-1-yle;
*N,N*-Diéthylcarbamate de 7-fluoro-2-(4-fluorophényl)-2*H*-benzo[*e*]indazol-1-yle;
Chlorhydrate de *N,N*-diéthylcarbamate de 7-chloro-2-(pyridin-4-yl)-4,5-dihydro-2*H-*benzo[*e*]indazol-1-yle ;
Chlorhydrate de *N,N*-diéthylcarbamate de 7-chloro-3-(pyridin-4-yl)-4,5-dihydro-3*H-*benzo[*e*]indazol-1-yle;
Chlorhydrate de *N,N-*diéthylcarbamate de 8-méthoxy-3-(pyridin-4-yl)-4,5-dihydro-3*H-*benzo[*e*]indazol-1-yle ;
*N,N-*Diméthylcarbamate de 2-(4-méthylphényl)-4,5-dihydro-2*H*-benzo[*e*]indazol-1-yle;
*N,N*-Diéthylcarbamate de 2-(4-chlorophényl)-4,5-dihydro-2*H*-benzo[*e*]indazol-1-yle;
Morpholinyl-4-carboxylate de 2-(3-chlorophényl)-4,5-dihydro-2*H*-benzo[*e*]indazol-1-yle;
*N,N*-Diméthylcarbamate de 2-(3-chlorophényl)-4,5-dihydro-2*H*-benzo[*e*]indazol-1-yle;
1*N*,*N*-Diéthylcarbamate de 2-(3-chlorophényl)-4,5-dihydro-2*H*-benzo[*e*]indazol-1-yle;
Pyrrolidinyl-1-carboxylate de 2-(3-chlorophényl)-4,5-dihydro-2*H*-benzo[*e*]indazol-1-yle ;
*N,N*-Diisopropylcarbamate de 2-(3-chlorophényl)-4,5-dihydro-2*H*-benzo[*e*]indazol-1-yle;
*N,N*-Diéthylcarbamate de 2-phényl-4,5-dihydro-2*H*-benzo[*e*]indazol-1-yle;
*N,N*-Diéthylcarbamate de 7-chloro-2-phényl-4,5-dihydro-2*H*-benzo[*e*]indazol-1-yle;
*N*-Méthyl-*N* -phénylcarbamate de 2-(3-chlorophényl)-4,5-dihydro-2*H*-benzo[*e*]indazol-1-yle;
*N,N*-Diéthylcarbamate de 2-(2-chlorophényl)-4,5-dihydro-2*H*-benzo[*e*]indazol-1-yle;
*N,N*-Diéthylcarbamate de 2-éthyl-4,5-dihydro-2*H*-benzo[*e*]indazol-1-yle;
*N*-Méthyl-*N*-phénylcarbamate de 2-éthyl-4,5-dihydro-2*H*-benzo[*e*]indazol -1-yle;
*N*-Méthyl-*N*-phénylcarbamate de 2-(2,2',2"-trifluoroéthyl)-4,5-dihydro-2*H*-benzo[*e*]indazol-1-yle;
*N*,*N*-Diéthylcarbamate de 2-(4-fluorophényl)-4,5-dihydro-2*H*-benzo[*e*]indazol-1-yle;
*N*,*N*-Diisopropylcarbamate de 2-(4-fluorophényl)-4,5-dihydro-2*H*-benzo[*e*]indazol-1-yle;
*N*-Méthyl-*N*-phénylcarbamate de 2-(4-fluorophényl)-4,5-dihydro-2*H*-benzo[*e*]indazol-1-yle;
Pyrrolidinyl-1-carboxylate de 2-(4-fluorophényl)-4,5-dihydro-2*H*-benzo[*e*]indazol-1-yle ;
Pipéridinyl-1-carboxylate de 2-(4-fluorophényl)-4,5-dihydro-2*H*-benzo[*e*]indazol-1-yle;
Morpholinyl-4-carboxylate de 2-(4-fluorophényl)-4,5-dihydro-2*H*-benzo[*e*]indazol-1-yle ;
*N,N*-Diméthylcarbamate de 2-(4-chlorophényl)-4,5-dihydro-2*H*-benzo[*e*]indazol-1-yle;
*N*,*N*-Diisopropylcarbamate de 2-(4-chlorophényl)-4,5-dihydro-2*H*-benzo[*e*]indazol-1-yle;
*N*-Méthyl-*N*-phénylcarbamate de 2-(4-chlorophényl)-4,5-dihydro-2*H*-benzo[*e*]indazol-1-yle;
Pyrrolidinyl-1-carboxylate de 2-(4-chlorophényl)-4,5-dihydro-2*H*-benzo[*e*]indazol-1-yle ;
Pipéridinyl-1-carboxylate de 2-(4-chlorophényl)-4,5-dihydro-2*H*-benzo[*e*]indazol-1-yle ;
Morpholinyl-4-carboxylate de 2-(4-chlorophényl)-4,5-dihydro-2*H*-benzo[*e*]indazol-1-yle ;
4-Méthyl-pipérazinyl-1-carboxylate de 2-(4-chlorophényl)-4,5-dihydro-2*H*-benzo[*e*]indazol-1-yle ;
*N,N*-Diméthylcarbamate de 2-(4-fluorophényl)-4,5-dihydro-2*H*-benzo[*e*]indazol-1-yle;
4-Méthyl-pipérazinyl-1-carboxylate de 2-(4-fluorophényl)-4,5-dihydro-2*H*-benzo[*e*]indazol-1-yle;
*N*,*N*-Diméthylcarbamate de 2-phényl-4,5-dihydro-2*H*-benzo[*e*]indazol-1-yle;
*N,N*-Disopropylcarbamate de 2-phényl-4,5-dihydro-2*H*-benzo[*e*]indazol-1-yle;
*N*-Méthyl-*N*-phénylcarbamate de 2-phényl-4,5-dihydro-2*H*-benzo[*e*]indazol-1-yle;
Pyrrolidinyl-1-carboxylate de 2-phényl-4,5-dihydro-2*H*-benzo[*e*]indazol-1-yle;
*N*,*N*-Diéthylcarbarmate de 7-chloro-2-(4-fluorophényl)-4,5-dihydro-2*H*-benzo[*e*]indazol-1-yle;
*N*-Méthyl-*N*-propylcarbamate de 2-(4-fluorophényl)-4,5-dihydro-2*H*-benzo[*e*]indazol-1-yle;
*N,N*-Diéthylcarbamate de 2-(3-chloro-4-méthylphényl)-4,5-dihydro-2*H*-benzo[*e*]indazol-1-yle;
*N*,*N*-Diéthylcarbamate de 2-(3-chloro-4-fluorophényl)-4,5-dihydro-2*H*-benzo[*e*]indazol-1-yle;
*N,N*-Diéthylcarbamate de 7-chloro-2-(3-chloro-4-méthylphényl)-4,5-dihydro-2*H-*benzo[*e*]indazol-1-yle;
*N*,*N*-Diéthylcarbamate de 7-chloro-2-(2,4-difluorophényl)-4,5-dihydro-2*H*-benzo[*e*]indazol-1-yle;
Chlorhydrate de N,N-diéthylcarbamate de 2-(pyridin-4-yl)-4,5-dihydro-2*H*-benzo[*e*]indazol-1-yle;
*N,N*-Diéthylcarbamate de 7-chloro-2-(pyridin-2-yl)-4,5-dihydro-2*H*-benzo[*e*]indazol-1-yle;
*N,N*-Diéthylcarbamate de 7-méthoxy-2-(4-fluorophényl)-4,5-dihydro-2*H*-benzo[*e*]indazol-1-yle;
*N,N*-Diéthylcarbamate de 7-bromo-2-(4-fluorophényl)-4,5-dihydro-2*H*-benzo[*e*]indazol-1-yle;
*N,N*-Diéthylcarbamate de 7-chloro-2-(3-chloro-4-fluorophényl)-4,5-dihydro-2*H*-benzo[*e*]indazol-1-yle;
*N,N*-Diéthylcarbamate de 7-chloro-2-méthyl-4,5-dihydro-2*H*-benzo[*e*]indazol-1-yle;
*N,N*-Diéthylcarbamate de 2-(3-chlorophényl)-2*H*-benzo[*e*]indazol-1-yle;
*N*,*N*-Diéthylcarbamate de 2-(4-fluorophényl)-2*H*-benzo[*e*]indazol-1-yle;
*N*-Méthyl-*N*-propylcarbamate de 2-(4-fluorophényl)-2*H*-benzo[*e*]indazol-1-yle;
*N,N*-Diéthylcarbamate de 2-(3-chloro-4-fluorophényl)-2*H*-benzo[*e*]indazol-1-yle;
*N,N*-Diéthylcarbamate de 2-(3-chloro-4-méthylphényl)-2*H*-benzo[*e*]indazol-1-yle;
*N,N*-Diéthylcarbamate de 7-chloro-2-(4-fluorophényl)-2*H*-benzo[*e*]indazol-1-yle;
*N,N*-Diéthylcarbamate de 7-chloro-2-(3-chloro-4-fluorophényl)-2*H*-benzo[*e*]indazol-1-yle;
*N,N*-Diéthylcarbamate de 7-chloro-2-(3-chloro-4-méthylphényl)-2*H*-benzo[*e*]indazol-1-yle;
*N,N*-Diéthylcarbamate de 7-chloro-2-(2,4-difluorophényl)-2*H*-benzo[*e*]indazol-1-yle;
Chlorhydrate de *N,N*-diéthylcarbamate de 7-méthoxy-3-(pyridin-4-yl)-4,5-dihydro-3*H-*benzo[*e*]indazol-1-yle;
Chlorhydrate de *N*,*N*-diéthylcarbamate de 7-méthyl-3-(pyridin-4-yl)-4,5-dihydro-3*H-*benzo[*e*]indazol-1-yle;
Chlorhydrate de *N,N*-diéthylcarbamate de 7-chloro-3-(pyridin-3-yl)-4,5-dihydro-3*H-*benzo[*e*]indazol-1-yle;
Chlorhydrate de *N*,*N*-diéthylcarbamate de 7-chloro-2-(pyridin-3-yl)-4,5-dihydro-2*H-*benzo[*e*]indazol-1-yle;
Chlorhydrate de *N,N*-diéthylcarbamate de 7-fluoro-2-(pyridin-4-yl)-4,5-dihydro-2*H-*benzo[*e*]indazol-1-yle;
Chlorhydrate de *N,N-*diéthylcarbamate de 7-fluoro-3-(pyridin-4-yl)-4,5-dihydro-3*H-*benzo[*e*]indazol-1-yle;
*N,N*-Diéthylcarbamate de 7-chloro-3-(4-fluorophényl)-4,5-dihydro-3*H*-benzo[*e*]indazol-1-yle;
Chlorhydrate de *N,N*-diéthylcarbamate de 7-chloro-3-(pyridin-2-yl)-4,5-dihydro-3*H-*benzo[*e*]indazol-1-yle;
*N,N*-Diéthylcarbamate de 7-chloro-2-(3-fluorophényl)-4,5-dihydro-2*H* benzo[*e*]indazol-1-yle;
*N,N*-Diéthylcarbamate de 7-chloro-3-(3-fluorophényl)-4,5-dihydro-3*H*-benzo[*e*]indazol-1-yle;
Chlorhydrate de *N,N-*diéthylcarbamate de 7-bromo-2-(pyridin-4-yl)-4,5-dihydro-2*H-*benzo[*e*]indazol-1-yle;
Chlorhydrate de *N,N-*diéthylcarbamate de 7-bromo-3-(pyridin-4-yl)-4,5-dihydro-3*H-*benzo[*e*]indazol-1-yle;
*N,N-*Diéthylcarbamate de 7-chloro-3-(4-méthylphényl)-4,5-dihydro-3*H*-benzo[*e*]indazol-1-yle;
*N,N*-Diéthylcarbamate de 7-chloro-2-(4-méthylphényl)-4,5-dihydro-2*H*-benzo[*e*]indazol-1-yle;
*N,N-*Diéthylcarbamate de 7-chloro-2-(2-fluorophényl)4,5-dihydro-2*H*-benzo[*e*]indazol-1-yle;
*N,N*-Diéthylcarbamate de 7-chloro-3-(4-chlorophényl)-4,5-dihydro-3*H*-benzo[*e*]indazol-1-yle;
*N,N*-Diéthylcarbamate de 7-chloro-2-(4-chlorophényl)-4,5-dihydro-2*H*-benzo[*e*]indazol-1-yle;
*N,N*-Diéthylcarbamate de 7-chloro-2-(4-méthoxyphényl)-4,5-dihydro-2*H*-benzo[*e*]indazol-1-yle;
*N,N*-Diéthylcarbamate de 7-chloro-3-(4-méthoxyphényl)-4,5-dihydro-3*H*-benzo[*e*]indazol-1-yle;
*N,N*-Diéthylcarbamate de 7-chloro-3-(3-fluorophényl)-3*H*-benzo[*e*]indazol-1-yle;
*N*-Méthyl-*N*-phénylcarbamate de 7-chloro-3-(pyridin-4-yl)-4,5-dihydro-3*H*-benzo[*e*]indazol-1-yle;
*N,N*-Diisopropylcarbamate de 7-chloro-2-(pyridin-4-yl)-4,5-dihydro-2*H*-benzo[*e*]indazol-1-yle;
Chlorhydrate de *N,N*-diisopropylcarbamate de 7-chloro-4,5-dihydro-3-(pyridin-4-yl)-3*H-*benzo[*e*]indazol-1-yle;
Chlorhydrate de *N,N*-diéthylcarbamate de 6-méthoxy-3-(pyridin-4-yl)-4,5-dihydro-3*H-*benzo[*e*]indazol-1-yle;
Chlorhydrate de *N,N*-diisopropylcarbamate de 7-chloro-3-(pyridin-4-yl)-3*H*-benzo[*e*]indazol-1-yle;
Chlorhydrate de pipéridinyl-1-carboxylate de 7-chloro-2-(pyridin-4-yl)-4,5-dihydro-2*H-*benzo[*e*]indazol-1-yle;
Chlorhydrate de pipéridinyl-1-carboxylate de 7-chloro-3-(pyridin-4-yl)-4,5-dihydro-3*H-*benzo[*e*]indazol-1-yle;
Chlorhydrate de *N,N*-diisopropylcarbamate de 7-chloro-2-(pyridin-3-yl)-4,5-dihydro-2*H-*benzo[*e*]indazol-1-yle;
Chlorhydrate de *N,N*-diisopropylcarbamate de 7-chloro-3-(pyridin-3-yl)-4,5-dihydro-3*H-*benzo[*e*]indazol-1-yle;
Chlorhydrate de *N,N*-diisopropylthiocarbamate de 7-chloro-3-(pyridin-4-yl)-4,5-dihydro-3*H-*benzo[*e*]indazol-1-yle;
Chlorhydrate de cis-2,6-diméthyl-pipéridinyl-1-carboxylate de 7-chloro-3-(pyridin-4-yl)-4,5-dihydro-3*H*-benzo[*e*]indazol-1-yle;
Chlorhydrate de *N*-méthyl-*N*-tertbutyl-carbamate de 7-chloro-2-(pyridin-4-yl)-4,5-dihydro-2*H-*benzo[*e*]indazol-1-yle;
Chlorhydrate de *N,N*-diisopropylcarbamate de 7-chloro-3-(pyridin-3-yl)-3*H*-benzo[*e*]indazol-1-yle;
*N,N*-Diisopropylcarbamate de 7-chloro-3-(pyridin-2-yl)-4,5-dihydro-3*H*-benzo[*e*]indazol-1-yle;
Chlorhydrate de *N,N*-diisopropylcarbamate de 7-fluoro-3-(pyridin-4-yl)-4,5-dihydro-3*H-*benzo[*e*]indazol-1-yle;
*N,N*-Diisopropylcarbamate de 7-chloro-3-(pyrimidin-2-yl)-4,5-dihydro-3*H*-benzo[*e*]indazol-1-yle;
*N,N*-Diisopropylcarbamate de 7-chloro-2-(pyrazin-2-yl)-4,5-dihydro-2*H*-benzo[*e*]indazol-1-yle;
Chlorhydrate de *N,N*-diisopropylcarbamate de 7-fluoro-3-(pyridin-4-yl)-3*H*-benzo[*e*]indazol-1-yle;
*N,N*-Diisopropylcarbamate de 7-chloro-3-(pyridin-2-yl)-3*H*-benzo[*e*]indazol-1-yle;
Chlorhydrate de *N*-méthyl-*N*-tertbutyl-carbamate de 7-chloro-3-(pyridin-4-yl)-4,5-dihydro-3*H-*benzo[*e*]indazol-1-yle;
Chlorhydrate de *N,N*-diéthylcarbamate de 7-chloro-3-(pyridin-4-yl)-3*H*-benzo[*e*]indazol-1-yle;
Chlorhydrate de *N*-méthyl-*N*-phénylcarbamate de 7-chloro-3-(pyridin-4-yl)-3*H*-benzo[*e*]indazol-1-yle.

6. Procédé de préparation d'un composé de formule (I) selon l'une quelconque des revendications 1 à 5, **caractérisé en ce que** l'on fait réagir
un mélange constitué des isomères de position de formules générales (Va) et (Vb), 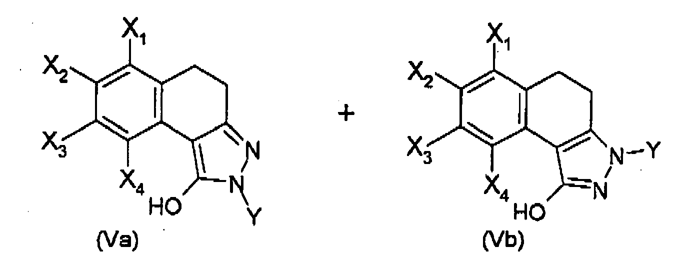 dans lesquelles X₁, X₂, X₃, X₄ et Y sont tels que définis dans la formule générale (I) selon la revendication 1 et dans lesquelles le groupe Y est respectivement en position (N2) et (N3) du cycle pyrazole,
avec un dérivé de chlorure de carbamoyle de formule générale ClC(W)NR₁R₂, dans laquelle W, R₁ et R₂ sont tels que définis dans la formule générale (I) selon la revendication 1, en présence d'une base,
pour obtenir, après séparation, les composés de formules générales (Ia) et (Ib), 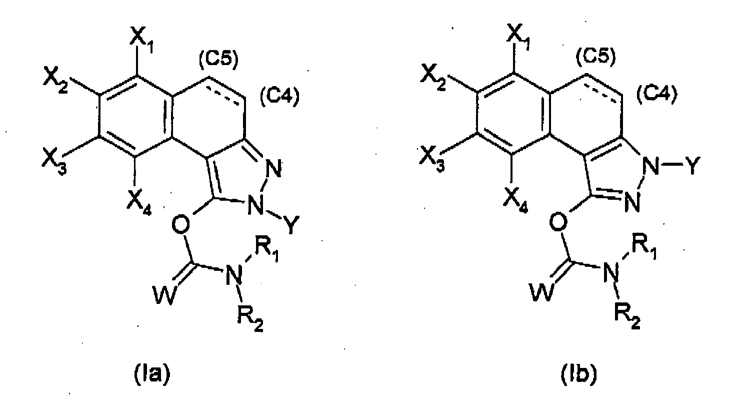 la liaison simple en position C4-C5 étant ensuite éventuellement déhydrogénée pour former une liaison double.

7. Procédé de préparation d'un composé de formule (Ia) 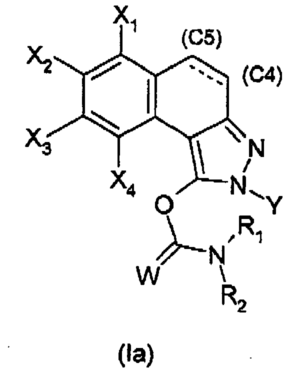 dans laquelle
W représente un atome d'oxygène ou de soufre ;
X₁, X₂, X₃ et X₄ représentent chacun, indépendamment l'un de l'autre, un atome d'hydrogène ou d'halogène ou un groupe cyano, C₁-C₆-alkyle, C₁-C₆-fluoroalkyle, C₁-C₆-alcoxy, C₁-C₆-fluoroalcoxy ;
Y est en position (N2) et représente un groupe C₁-C₆-alkyle, C₁-C₆-fluoroalkyle, aryle comprenant entre 6 et 10 atomes de carbone ou hétéroaryle comprenant de 5 à 6 chaînons et comprenant entre 1 et 2 hétéroatomes;
les groupes aryle ou hétéroaryle étant éventuellement substitués par un ou plusieurs atomes ou groupes choisis parmi les atomes d'halogène, les groupes C₁-C₆-alkyle, C₁-C₆-alcoxy, C₁-C₆-alkylthio, C₁-C₆-alkyl-S(O)-, C₁-C₆-alkyl-S(O)₂-, C₁-C₆-fluoroalkyle ;
la liaison en position C4-C5 est double ou simple ;
R₁ et R₂ représentent chacun, indépendamment l'un de l'autre, un groupe aryle comprenant entre 6 et 10 atomes de carbone, benzyle ou C₁-C₆-alkyle ; ou bien R₁ et R₂ forment, avec l'atome d'azote qui les porte un hétérocycle, comprenant de 4 à 7 chaînons et comprenant un atome d'azote et éventuellement un autre hétéroatome, éventuellement substitué par un groupe C₁-C₆-alkyle où benzyle ;
**caractérisé en ce que** l'on fait réagir le composé de formule générale (Va), 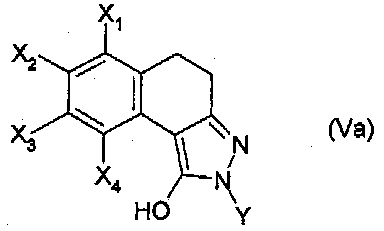 dans laquelle X₁, X₂, X₃, X₄ et Y sont tels que définis dans la formule générale (la) ci-dessus, avec un dérivé de chlorure de carbamoyle de formule générale ClC(W)NR₁R₂, dans laquelle W, R₁ et R₂ sont tels que définis dans la formule générale (la) ci-dessus, en présence d'une base,
la liaison simple en position C4-C5 étant ensuite éventuellement déhydrogénée pour former une liaison double.

8. Composé de formule (Va) 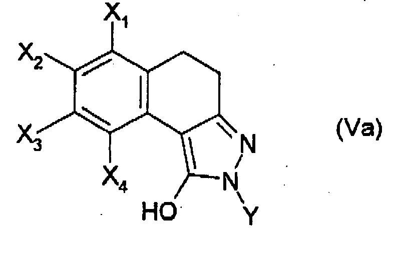 dans laquelle
X₁, X₂, X₃ et X₄ représentent chacun, indépendamment l'un de l'autre, un atome d'hydrogène ou d'halogène ou un groupe cyano, C₁-C₆-alkyle, C₁-C₆-fluoroalkyle, C₁-C₆-alcoxy, C₁-C₆-fluoroalcoxy ;
Y représente un groupe C₁-C₆-alkyle, C₁-C₆-fluoroalkyle, aryle comprenant entre 6 et 10 atomes de carbone ou hétéroaryle comprenant de 5 à 6 chaînons et comprenant entre 1 et 2 hétéroatomes;
les groupes aryle ou hétéroaryle étant éventuellement substitués par un ou plusieurs atomes ou groupes choisis parmi les atomes d'halogène, les groupes C₁-C₆-alkyle, C₁-C₆-alcoxy, C₁-C₆-alkylthio, C₁-C₆-alkyl-S(O)-, C₁-C₆-alkyl-S(O)₂-, C₁-C₆-fluoroalkyle.

9. Composé de formule (Vb) 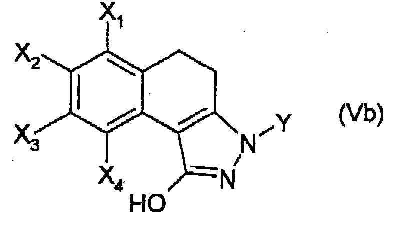 dans laquelle
X₁, X₂, X₃ et X₄ représentent chacun, indépendamment l'un de l'autre, un atome d'hydrogène ou d'halogène ou un groupe cyano, C₁-C₆-alkyle, C₁-C₆-fluoroalkyle, C₁-C₆-alcoxy, C₁-C₆-fluoroalcoxy ;
Y représente un groupe aryle comprenant entre 6 et 10 atomes de carbone ou hétéroaryle comprenant de 5 à 6 chaînons et comprenant entre 1 et 2 hétéroatomes;
les groupes aryle ou hétéroaryle étant éventuellement substitués par un ou plusieurs atomes ou groupes choisis parmi les atomes d'halogène, les groupes C₁-C₆-alkyle, C₁-C₆-alcoxy, C₁-C₆-alkylthio, C₁-C₆-alkyl-S(O)-, C₁-C₆-alkyl-S(O)₂-, C₁-C₆-fluoroalkyle.

10. Médicament, **caractérisé en ce qu'**il comprend un composé de formule (I) selon l'une quelconque des revendications 1 à 5, ou un sel d'addition de ce composé à un acide pharmaceutiquement acceptable, ou encore un hydrate ou un solvate du composé de formule (I).

11. Composition pharmaceutique, **caractérisée en ce qu'**elle comprend un composé de formule (I) selon l'une quelconque des revendications 1 à 5, ou un sel pharmaceutiquement acceptable, un hydrate ou un solvate de ce composé, ainsi qu'au moins un excipient pharmaceutiquement acceptable.

12. Utilisation d'un composé de formule (I) selon l'une quelconque des revendications 1 à 5 pour la préparation d'un médicament destiné à prévenir ou à traiter les neuropathies périphériques, les affections du motoneurone, les maladies neurodégénératives du système nerveux central, l'anxiété, l'épilepsie, les troubles du sommeil, l'insuffisance rénale aiguë ou chronique, la glomérulonéphrite, la néphropathie diabétique, l'ischémie et l'insuffisance cardiaques, l'infarctus du myocarde, l'ischémie des membres inférieurs, le vasospasme coronaire, l'angine de poitrine, les pathologies associées aux valves cardiaques, les maladies cardiaques inflammatoires, les effets secondaires dus à des médicaments cardiotoxiques ou aux suites d'une chirurgie cardiaque, l'athérosclérose et ses complications thrombo-emboliques, la resténose, les rejets de greffes, les conditions liées à une prolifération ou une migration incorrectes des cellules musculaires lisses, les tumeurs et cancers, les stress cutanés, les maladies inflammatoires chroniques, notamment la polyarthrite rhumatoïde, et les maladies inflammatoires pulmonaires.

13. Utilisation d'un composé de formule (I) selon la revendication 12, où les neuropathies périphériques sont choisies parmi les neuropathies traumatiques ou ischémiques, les neuropathies infectieuses, alcooliques, diabétiques, médicamenteuses ou génétiques.

14. Utilisation d'un composé de formule (I) selon la revendication 12, où les affections du motoneurone sont choisies parmi les amyotrophies spinales et la sclérose latérale amyotrophique.

15. Utilisation d'un composé de formule (I) selon la revendication 12, où les maladies neurodégénératives du système nerveux central sont choisies parmi les accidents vasculaires cérébraux, les traumatismes crâniens et médullaires, la sclérose en plaques, la maladie d'Alzheimer, ou la maladie de Parkinson.

## Claims

1. Compound corresponding to the formula (I) 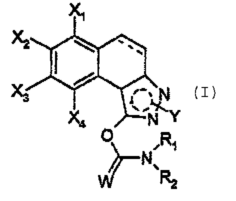 in which
W represents an oxygen or sulphur atom;
X₁, X₂, X₃ and X₄ each represent, independently of one another, a hydrogen or halogen atom or a cyano, C₁-C₆-alkyl, C₁-C₆-fluoroalkyl, C₁-C₆-alkoxy or C₁-C₆-fluoroalkoxy group;
Y is in the (N2) or (N3) position;
when Y is in the (N2) position, Y represents a C₁-C₆-alkyl group, a C₁-C₆-fluoroalkyl group, an aryl group comprising between 6 and 10 carbon atoms or a heteroaryl group comprising from 5 to 6 ring members and comprising between 1 and 2 heteroatoms;
when Y is in the (N3) position, Y represents an aryl or heteroaryl group;
the aryl or heteroaryl groups optionally being substituted by one or more atoms or groups chosen from halogen atoms or C₁-C₆-alkyl, C₁-C₆-alkoxy, C₁-C₆-alkylthio, C₁-C₆-alkyl-S(O)-, C₁-C₆-alkyl-S(O)₂- or C₁-C₆-fluoroalkyl groups;
the bond in the C4-C5 position is a double or single bond;
R₁ and R₂ each represent, independently of one another, an aryl group comprising between 6 and 10 carbon atoms, a benzyl group or a C₁-C₆-alkyl group; or else R₁ and R₂ form, with the nitrogen atom which carries them, a heterocycle, comprising from 4 to 7 ring members and comprising a nitrogen atom and optionally another heteroatom, optionally substituted by one or more C₁-C₆-alkyl or benzyl groups;
in the form of the base or of an addition salt with an acid, and in the hydrate or solvate form.

2. Compound of formula (I) according to Claim 1, **characterized in that**
W represents an oxygen or sulphur atom;
X₁, X₂ and X₃ each represent, independently of one another, a hydrogen atom, a halogen atom, a cyano group, a C₁-C₆-alkyl group or a C₁-C₆-alkoxy group;
X₄ represents a hydrogen atom;
Y is in the (N2) or (N3) position;
when Y is in the (N2) position, Y represents a C₁-C₆-alkyl group, a C₁-C₆-fluoroalkyl group, an aryl group comprising between 6 and 10 carbon atoms or a heteroaryl group comprising from 5 to 6 ring members and comprising between 1 and 2 heteroatoms;
when Y is in the (N3) position, Y represents an aryl group or a heteroaryl group;
the aryl or heteroaryl groups optionally being substituted by one or more atoms or groups chosen from halogen atoms, C₁-C₆-alkyl groups and C₁-C₆-alkoxy groups;
the bond in the C4-C5 position is a double or single bond;
R₁ and R₂ each represent, independently of one another, an aryl group comprising between 6 and 10 carbon atoms or a C₁-C₆-alkyl group; or else R₁ and R₂ form, with the nitrogen atom which carries them, a heterocycle, comprising from 4 to 7 ring members and comprising a nitrogen atom and optionally another heteroatom, optionally substituted by one or two C₁-C₆-alkyl groups; in the form of the base or of an addition salt with an acid, and in the hydrate or solvate form.

3. Compound of formula (I) according to Claim 1 or 2, **characterized in that**
W represents an oxygen or sulphur atom;
X₁, X₂ and X₃ each represent, independently of one another, a hydrogen atom, a halogen atom, a C₁-C₆-alkyl group or a C₁-C₆-alkoxy group;
X₄ represents a hydrogen atom;
Y is in the (N2) or (N3) position and represents an aryl group comprising between 6 and 10 carbon atoms or a heteroaryl group comprising from 5 to 6 ring members and comprising between 1 and 2 heteroatoms;
the aryl or heteroaryl groups optionally being substituted by one or more atoms or groups chosen from halogen atoms, C₁-C₆-alkyl groups and C₁-C₆-alkoxy groups;
the bond in the C4-C5 position is a double or single bond;
R₁ and R₂ each represent, independently of one another, an aryl group comprising between 6 and 10 carbon atoms or a C₁-C₆-alkyl group; or else R₁ and R₂ form, with the nitrogen atom which carries them, a heterocycle, comprising from 4 to 7 ring members and comprising a nitrogen atom and optionally another heteroatom, optionally substituted by one or two C₁-C₆-alkyl groups; in the form of the base or of an addition salt with an acid, and in the hydrate or solvate form.

4. Compound of formula (I) according to any one of Claims 1 to 3, **characterized in that**
W represents an oxygen or sulphur atom;
X₁, X₂ and X₃ each represent, independently of one another, a hydrogen atom, a halogen atom, a C₁-C₆-alkyl group or a C₁-C₆-alkoxy group;
X₄ represents a hydrogen atom;
Y is in the (N3) position and represents an aryl group comprising between 6 and 10 carbon atoms or a heteroaryl group comprising from 5 to 6 ring members and comprising between 1 and 2 heteroatoms;
the aryl or heteroaryl groups optionally being substituted by one or more atoms or groups chosen from halogen atoms, C₁-C₆-alkyl groups and C₁-C₆-alkoxy groups;
the bond in the C4-C5 position is a double or single bond;
R₁ and R₂ each represent, independently of one another, an aryl group comprising between 6 and 10 carbon atoms or a C₁-C₆-alkyl group; or else R₁ and R₂ form, with the nitrogen atom which carries them, a heterocycle, comprising from 4 to 7 ring members and comprising a nitrogen atom and optionally another heteroatom, optionally substituted by one or two C₁-C₆-alkyl groups; in the form of the base or of an addition salt with an acid, and in the hydrate or solvate form.

5. Compound of formula (I) according to any one of Claims 1 to 4, **characterized in that** it is chosen from:
7-Fluoro-2-(4-fluorophenyl)-4,5-dihydro-2*H-*benzo[*e*]indazol-1-yl *N,N*-diethylcarbamate;
7-Fluoro-2-(4-fluorophenyl)-2*H*-benzo[*e*]indazol-1-yl *N,N*-diethylcarbamate;
7-Chloro-2-(pyridin-4-yl)-4,5-dihydro-2*H*-benzo[*e*]-indazol-1-yl *N,N*-diethylcarbamate hydrochloride;
7-Chloro-3-(pyridin-4-yl)-4,5-dihydro-3*H*-benzo[*e*]-indazol-1-yl *N*,*N*-diethylcarbamate hydrochloride;
8-Methoxy-3-(pyridin-4-yl)-4,5-dihydro-3*H*-benzo[*e*]-indazol-1-yl *N,N*-diethylcarbamate hydrochloride;
2-(4-Methylphenyl)-4,5-dihydro-2*H*-benzo[*e*]indazol-1-yl *N,N*-dimethylcarbamate;
2-(4-Chlorophenyl)-4,5-dihydro-2*H*-benzo[*e*]indazol-1-yl *N,N*-diethylcarbamate;
2-(3-Chlorophenyl)-4,5-dihydro-2*H*-benzo[*e*]indazol-1-yl morpholine-4-carboxylate;
2-(3-Chlorophenyl)-4,5-dihydro-2*H*-benzo[*e*]indazol-1-yl *N,N*-dimethylcarbamate;
2-(3-Chlorophenyl)-4,5-dihydro-2*H*-benzo[*e*]indazol-1-yl *N,N*-diethylcarbamate;
2-(3-Chlorophenyl)-4,5-dihydro-2*H*-benzo[*e*]indazol-1-yl pyrrolidine-1-carboxylate;
2-(3-Chlorophenyl)-4,5-dihydro-2*H*-benzo[*e*]indazol-1-yl *N*,*N*-diisopropylcarbamate;
2-Phenyl-4,5-dihydro-2*H*-benzo[e]indazol-1-yl *N*,*N*-di-ethylcarbamate;
7-Chloro-2-phenyl-4,5-dihydro-2*H*-benzo[*e*]indazol-1-yl *N,N*-diethylcarbamate;
2-(3-Chlorophenyl)-4,5-dihydro-2*H*-benzo[*e*]indazol-1-yl *N*-methyl-*N*-phenylcarbamate;
2-(2-Chlorophenyl)-4,5-dihydro-2*H*-benzo[*e*]indazol-1-yl *N,N*-diethylcarbamate;
2-Ethyl-4,5-dihydro-2*H*-benzo[*e*]indazol-1-yl *N*,*N*-di-ethylcarbamate;
2-Ethyl-4,5-dihydro-2*H*-benzo[*e*]indazol-1-yl *N*-methyl-*N*-phenylcarbamate;
2-(2,2',2"-Trifluoroethyl)-4,5-dihydro-2*H*-benzo[*e*]-indazol-1-yl *N*-methyl-*N*-phenylcarbamate;
2-(4-Fluorophenyl)-4,5-dihydro-2*H*-benzo[*e*]indazol-1-yl *N,N*-diethylcarbamate;
2-(4-Fluorophenyl)-4,5-dihydro-2*H*-benzo[*e*]indazol-1-yl *N,N*-diisopropylcarbamate;
2-(4-Fluorophenyl)-4,5-dihydro-2*H*-benzo[*e*]indazol-1-yl *N*-methyl-*N*-phenylcarbamate;
2-(4-Fluorophenyl)-4,5-dihydro-2*H*-benzo[*e*]indazol-1-yl pyrrolidine-1-carboxylate;
2-(4-Fluorophenyl)-4,5-dihydro-2*H*-benzo[*e*]indazol-1-yl piperidine-1-carboxylate;
2-(4-Fluorophenyl)-4,5-dihydro-2*H*-benzo[*e*]indazol-1-yl morpholine-4-carboxylate;
2-(4-Chlorophenyl)-4,5-dihydro-2*H*-benzo[*e*]indazol-1-yl *N,N*-dimethylcarbamate;
2-(4-Chlorophenyl)-4,5-dihydro-2*H*-benzo[*e*]indazol-1-yl *N,N*-diisopropylcarbamate;
2-(4-Chlorophenyl)-4,5-dihydro-2*H*-benzo[*e*]indazol-1-yl *N*-methyl-*N*-phenylcarbamate;
2-(4-Chlorophenyl)-4,5-dihydro-2*H*-benzo[*e*]indazol-1-yl pyrrolidine-1-carboxylate;
2-(4-Chlorophenyl)-4,5-dihydro-2*H*-benzo[*e*]indazol-1-yl piperidine-1-carboxylate;
2-(4-Chlorophenyl)-4,5-dihydro-2*H*-benzo[*e*]indazol-1-yl morpholine-4-carboxylate;
2-(4-Chlorophenyl)-4,5-dihydro-2*H*-benzo[*e*]indazol-1-yl 4-methylpiperazine-1-carboxylate;
2-(4-Fluorophenyl)-4,5-dihydro-2*H*-benzo[*e*]indazol-1-yl *N*,*N*-dimethylcarbamate;
2-(4-Fluorophenyl)-4,5-dihydro-2*H*-benzo[*e*]indazol-1-yl 4-methylpiperazine-1-carboxylate;
2-Phenyl-4,5-dihydro-2*H*-benzo[*e*]indazol-1-yl *N,N*-dimethylcarbamate;
2-Phenyl-4,5-dihydro-2*H*-benzo[*e*]indazol-1-yl *N,N*-diisopropylcarbamate;
2-Phenyl-4,5-dihydro-2*H*-benzo[*e*]indazol-1-yl *N*-methyl-*N*-phenylcarbamate;
2-Phenyl-4,5-dihydro-2*H*-benzo[*e*]indazol-1-yl pyrrolidine-1-carboxylate;
7-Chloro-2-(4-fluorophenyl)-4,5-dihydro-2*H*-benzo[*e*]-indazol-1-yl *N,N*-diethylcarbamate;
2-(4-Fluorophenyl)-4,5-dihydro-2*H*-benzo[*e*]indazol-1-yl *N*-methyl-*N*-propylcarbamate;
2-(3-Chloro-4-methylphenyl)-4,5-dihydro-2*H*-benzo[*e*]-indazol-1-yl *N,N*-diethylcarbamate;
2-(3-Chloro-4-fluorophenyl)-4,5-dihydro-2*H*-benzo[*e*]-indazol-1-yl *N,N*-diethylcarbamate;
7-Chloro-2-(3-chloro-4-methylphenyl)-4,5-dihydro-2*H-*benzo[*e*]indazol-1-yl *N,N*-diethylcarbamate;
7-Chloro-2-(2,4-difluorophenyl)-4,5-dihydro-2*H-*benzo[*e*]indazol-1-yl *N,N*-diethylcarbamate;
2-(Pyridin-4-yl)-4,5-dihydro-2*H*-benzo[*e*]indazol-1-yl *N,N*-diethylcarbamate hydrochloride;
7-Chloro-2-(pyridin-2-yl)-4,5-dihydro-2*H*-benzo[*e*]-indazol-1-yl *N,N*-diethylcarbamate;
7-Methoxy-2-(4-fluorophenyl)-4,5-dihydro-2*H*-benzo[*e*]-indazol-1-yl *N,N*-diethylcarbamate;
7-Bromo-2-(4-fluorophenyl)-4,5-dihydro-2*H*-benzo[*e*]-indazol-1-yl *N,N*-diethylcarbamate;
7-Chloro-2-(3-chloro-4-fluorophenyl)-4,5-dihydro-2*H-*benzo[*e*]indazol-1-yl *N,N*-diethylcarbamate;
7-Chloro-2-methyl-4,5-dihydro-2*H*-benzo[*e*]indazol-1-yl *N,N*-diethylcarbamate;
2-(3-Chlorophenyl)-2*H*-benzo[*e*]indazol-1-yl *N,N*-diethylcarbamate;
2-(4-Fluorophenyl)-2*H*-benzo[*e*]indazol-1-yl *N,N*-diethylcarbamate;
2-(4-Fluorophenyl)-2*H*-benzo[*e*]indazol-1-yl *N*-methyl-*N*-propylcarbamate;
2-(3-Chloro-4-fluorophenyl)-2*H*-benzo[*e*]indazol-1-yl *N,N*-diethylcarbamate;
2-(3-Chloro-4-methylphenyl)-2*H*-benzo[*e*]indazol-1-yl *N,N*-diethylcarbamate;
7-Chloro-2-(4-fluorophenyl)-2*H*-benzo[*e*]indazol-1-yl *N,N*-diethylcarbamate;
7-Chloro-2-(3-chloro-4-fluorophenyl)-2*H*-benzo[*e*]-indazol-1-yl *N,N*-diethylcarbamate;
7-Chloro-2-(3-chloro-4-methylphenyl)-2*H*-benzo[*e*]-indazol-1-yl *N,N*-diethylcarbamate;
7-Chloro-2-(2,4-difluorophenyl)-2*H*-benzo[*e*]indazol-1-yl *N,N*-diethylcarbamate;
7-Methoxy-3-(pyridin-4-yl)-4,5-dihydro-3*H*-benzo[*e*]-indazol-1-yl *N,N*-diethylcarbamate hydrochloride;
7-Methyl-3-(pyridin-4-yl)-4,5-dihydro-3*H*-benzo[*e*]-indazol-1-yl *N,N*-diethylcarbamate hydrochloride;
7-Chloro-3-(pyridin-3-yl)-4,5-dihydro-3*H*-benzo[*e*]-indazol-1-yl *N,N*-diethylcarbamate hydrochloride;
7-Chloro-2-(pyridin-3-yl)-4,5-dihydro-2*H*-benzo[*e*]-indazol-1-yl *N,N*-diethylcarbamate hydrochloride;
7-Fluoro-2-(pyridin-4-yl)-4,5-dihydro-2*H*-benzo[*e*]-indazol-1-yl *N,N*-diethylcarbamate hydrochloride;
7-Fluoro-3-(pyridin-4-yl)-4,5-dihydro-3*H*-benzo[*e*]-indazol-1-yl *N,N*-diethylcarbamate hydrochloride;
7-Chloro-3-(4-fluorophenyl)-4,5-dihydro-3*H*-benzo[*e*]-indazol-1-yl *N,N*-diethylcarbamate;
7-Chloro-3-(pyridin-2-yl)-4,5-dihydro-3*H*-benzo[*e*]-indazol-1-yl *N,N*-diethylcarbamate hydrochloride;
7-Chloro-2-(3-fluorophenyl)-4,5-dihydro-2*H*-benzo[*e*]-indazol-1-yl *N,N*-diethylcarbamate;
7-Chloro-3-(3-fluorophenyl)-4,5-dihydro-3*H*-benzo[*e*]-indazol-1-yl *N,N*-diethylcarbamate;
7-Bromo-2-(pyridin-4-yl)-4,5-dihydro-2*H*-benzo[*e*]-indazol-1-yl *N,N*-diethylcarbamate hydrochloride;
7-Bromo-3-(pyridin-4-yl)-4,5-dihydro-3*H*-benzo[*e*]-indazol-1-yl *N,N*-diethylcarbamate hydrochloride;
7-Chloro-3-(4-methylphenyl)-4,5-dihydro-3*H*-benzo[*e*]-indazol-1-yl *N,N*-diethylcarbamate;
7-Chloro-2-(4-methylphenyl)-4,5-dihydro-2*H*-benzo[*e*]-indazol-1-yl *N,N*-diethylcarbamate;
7-Chloro-2-(2-fluorophenyl)-4,5-dihydro-2*H*-benzo[*e*]-indazol-1-yl *N,N*-diethylcarbamate;
7-Chloro-3-(4-chlorophenyl)-4,5-dihydro-3*H*-benzo[*e*]-indazol-1-yl *N,N*-diethylcarbamate;
7-Chloro-2-(4-chlorophenyl)-4,5-dihydro-2*H*-benzo[*e*]-indazol-1-yl *N,N*-diethylcarbamate;
7-Chloro-2-(4-methoxyphenyl)-4,5-dihydro-2*H*-benzo[*e*]-indazol-1-yl *N,N*-diethylcarbamate;
7-Chloro-3-(4-methoxyphenyl)-4,5-dihydro-3*H*-benzo[*e*]-indazol-1-yl *N,N*-diethylcarbamate;
7-Chloro-3-(3-fluorophenyl)-3*H*-benzo[*e*]indazol-1-yl *N,N*-diethylcarbamate;
7-Chloro-3-(pyridin-4-yl)-4,5-dihydro-3*H*-benzo[*e*]-indazol-1-yl *N*-methyl-*N*-phenylcarbamate:
7-Chloro-2-(pyridin-4-yl)-4,5-dihydro-2*H*-benzo[*e*]-indazol-1-yl *N,N*-diisopropylcarbamate;
7-Chloro-4,5-dihydro-3-(pyridin-4-yl)-3*H*-benzo[*e*]-indazol-1-yl *N,N*-diisopropylcarbamate hydrochloride;
6-Methoxy-3-(pyridin-4-yl)-4,5-dihydro-3*H*-benzo[*e*]-indazol-1-yl *N,N*-diethylcarbamate hydrochloride;
7-Chloro-3-(pyridin-4-yl)-3*H*-benzo[e]indazol-1-yl *N,N*-diisopropylcarbamate hydrochloride;
7-Chloro-2-(pyridin-4-yl)-9,5-dihydro-2*H*-benzo[*e*]-indazol-1-yl piperidine-1-carboxylate hydrochloride;
7-Chloro-3-(pyridin-9-yl)-4,5-dihydro-3*H*-benzo[*e*]-indazol-1-yl piperidine-1-carboxylate hydrochloride;
7-Chloro-2-(pyridin-3-yl)-4,5-dihydro-2*H*-benzo[*e*]-indazol-1-yl *N,N*-diisopropylcarbamate hydrochloride;
7-Chloro-3-(pyridin-3-yl)-4,5-dihydro-3*H*-benzo[*e*]-indazol-1-yl *N,N*-diisopropylcarbamate hydrochloride;
7-Chloro-3-(pyridin-4-yl)-4,5-dihydro-3*H*-benzo[*e*]-indazol-1-yl *N,N*-diisopropylthiocarbamate hydrochloride;
7-Chloro-3-(pyridin-4-yl)-4,5-dihydro-3*H*-benzo[*e*]-indazol-1-yl cis-2,6-dimethylpiperidine-1-carboxylate hydrochloride;
7-Chloro-2-(pyridin-4-yl)-4,5-dihydro-2*H*-benzo[*e*]-indazol-1-yl *N*-methyl-*N*-(tert-butyl)carbamate hydrochloride;
7-Chloro-3-(pyridin-3-yl)-3*H*-benzo[*e*]indazol-1-yl *N,N-*diisopropylcarbamate hydrochloride;
7-Chloro-3-(pyridin-2-yl)-4,5-dihydro-3*H*-benzo[*e*]-indazol-1-yl *N,N*-diisopropylcarbamate;
7-Fluoro-3-(pyridin-4-yl)-4,5-dihydro-3*H*-benzo[*e*]-indazol-1-yl *N,N*-diisopropylcarbamate hydrochloride;
7-Chloro-3-(pyrimidin-2-yl)-4,5-dihydro-3*H*-benzo[*e*]-indazol-1-yl *N,N*-diisopropylcarbamate;
7-Chloro-2-(pyrazin-2-yl)-4,5-dihydro-2*H*-benzo[*e*]-indazol-1-yl *N,N*-diisopropylcarbamate;
7-Fluoro-3-(pyridin-4-yl)-3*H*-benzo[*e*]indazol-1-yl *N,N-*diisopropylcarbamate hydrochloride;
7-Chloro-3-(pyridin-2-yl)-3*H*-benzo[*e*]indazol-1-yl *N,N-*diisopropylcarbamate;
7-Chloro-3-(pyridin-4-yl)-4,5-dihydro-3*H*-benzo[*e*]-indazol-1-yl *N*-methyl-*N*-(tert-butyl)carbamate hydrochloride;
7-Chloro-3-(pyridin-4-yl)-3*H*-benzo[*e*]indazol-1-yl *N,N*-diethylcarbamate hydrochloride;
7-Chloro-3-(pyridin-4-yl)-3*H*-benzo[*e*]indazol-1-yl *N-*methyl-*N*-phenylcarbamate hydrochloride;

6. Process for the preparation of a compound of formula (I) according to any one of Claims 1 to 5,
**characterized in that**
a mixture composed of the positional isomers of general formulae (Va) and (Vb), 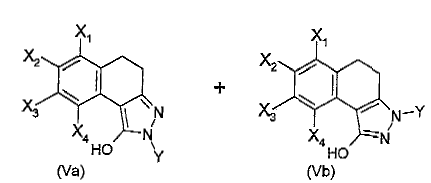 in which X₁, X₂, X₃, X₄ and Y are as defined in the general formula (I) according to Claim 1 and in which the Y group is respectively in the (N2) position and in the (N3) position of the pyrazole ring,
is reacted with a carbamoyl chloride derivative of general formula ClC(W)NR₁R₂, in which W, R₁ and R₂ are as defined in the general formula (I) according to Claim 1, in the presence of a base,
to obtain, after separation, the compounds of general formulae (Ia) and (Ib), 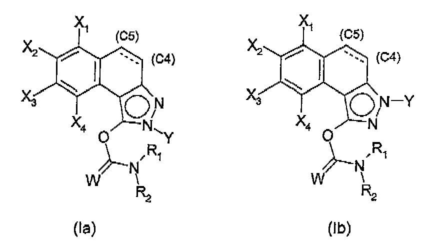 the single bond in the C4-C5 position subsequently being optionally dehydrogenated to form a double bond.

7. Process for the preparation of a compound of formula (Ia) 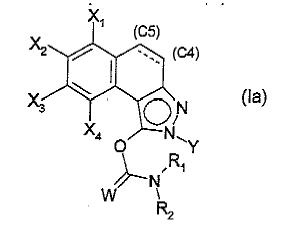 in which
W represents an oxygen or sulphur atom;
X₁, X₂, X₃ and X₄ each represent, independently of one another, a hydrogen or halogen atom or a cyano, C₁-C₆-alkyl, C₁-C₆-fluoroalkyl, C₁-C₆-alkoxy or C₁-C₆-fluoroalkoxy group;
Y is in the (N2) position and represents a C₁-C₆-alkyl group, a C₁-C₆-fluoroalkyl group, an aryl comprising between 6 and 10 carbon atoms or a heteroaryl group comprising from 5 to 6 ring members and comprising between 1 and 2 heteroatoms;
the aryl or heteroaryl groups optionally being substituted by one or more atoms or groups chosen from halogen atoms or C₁-C₆-alkyl, C₁-C₆-alkoxy, C₁-C₆-alkylthio, C₁-C₆-alkyl-S(O)-, C₁-C₆-alkyl-S(O)₂- or C₁-C₆-fluoroalkyl groups;
the bond in the C4-C5 position is a double or single bond;
R₁ and R₂ each represent, independently of one another, an aryl group comprising between 6 and 10 carbon atoms, a benzyl group or a C₁-C₆-alkyl group; or else R₁ and R₂ form, with the nitrogen atom which carries them, a heterocycle, comprising from 4 to 7 ring members and comprising a nitrogen atom and optionally another heteroatom, optionally substituted by a C₁-C₆-alkyl or benzyl group;
**characterized in that** the compound of general formula (Va), 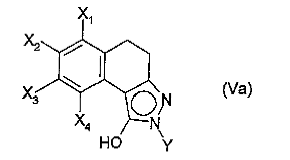 in which X₁, X₂, X₃, X₄ and Y are as defined in the general formula (Ia) above,
is reacted with a carbamoyl chloride derivative of general formula ClC(W)NR₁R₂, in which W, R₁ and R₂ are as defined in the general formula (Ia) above, in the presence of a base,
the single bond in the C4-C5 position subsequently being optionally dehydrogenated to form a double bond.

8. Compound of formula (Va) 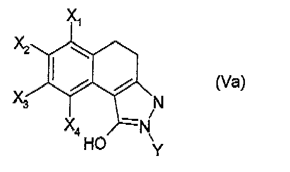 in which
X₁, X₂, X₃ and X₄ each represent, independently of one another, a hydrogen or halogen atom or a cyano, C₁-C₆-alkyl, C₁-C₆-fluoroalkyl, C₁-C₆-alkoxy or C₁-C₆-fluoroalkoxy group;
Y represents a C₁-C₆-alkyl group, a C₁-C₆-fluoroalkyl group, an aryl group comprising between 6 and 10 carbon atoms or a heteroaryl group comprising from 5 to 6 ring members and comprising between 1 and 2 heteroatoms;
the aryl or heteroaryl groups optionally being substituted by one or more atoms or groups chosen from halogen atoms or C₁-C₆-alkyl, C₁-C₆-alkoxy, C₁-C₆-alkylthio, C₁-C₆-alkyl-S(O)-, C₁-C₆-alkyl-S(O)₂- or C₁-C₆-fluoroalkyl groups.

9. Compound of formula (Vb) 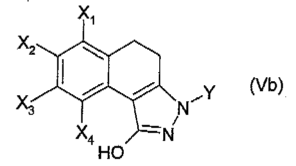 in which
X₁, X₂, X₃ and X₄ each represent, independently of one another, a hydrogen or halogen atom or a cyano, C₁-C₆-alkyl, C₁-C₆-fluoroalkyl, C₁-C₆-alkoxy or C₁-C₆-fluoroalkoxy group;
Y represents an aryl group comprising between 6 and 10 carbon atoms or a heteroaryl group comprising from 5 to 6 ring members and comprising between 1 and 2 heteroatoms;
the aryl or heteroaryl groups optionally being substituted by one or more atoms or groups chosen from halogen atoms or C₁-C₆-alkyl, C₁-C₆-alkoxy, C₁-C₆-alkylthio, C₁-C₆-alkyl-S(O)-, C₁-C₆-alkyl-S(O)₂- or C₁-C₆-fluoroalkyl groups.

10. Medicament, **characterized in that** it comprises a compound of formula (I) according to any one of Claims 1 to 5, or an addition salt of this compound with a pharmaceutically acceptable acid, or a hydrate or a solvate of the compound of formula (I).

11. Pharmaceutical composition, **characterized in that** it comprises a compound of formula (I) according to any one of Claims 1 to 5, or a pharmaceutically acceptable salt, a hydrate or a solvate of this compound, and at least one pharmaceutically acceptable excipient.

12. Use of a compound of formula (I) according to any one of Claims 1 to 5 for the preparation of a medicament intended to prevent or treat peripheral neuropathies, motor neuron conditions, neurodegenerative diseases of the central nervous system, anxiety, epilepsy, sleep disorders, acute or chronic renal insufficiency, glomerulonephritis, diabetic nephropathy, cardiac ischaemia and cardiac insufficiency, myocardial infarction, ischaemia of the lower limbs, coronary vasospasm, angina pectoris, pathologies associated with the heart valves, inflammatory heart diseases, side effects due to cardiotoxic medicaments or as a result of heart surgery, atherosclerosis and its thromboembolic complications, restenosis, graft rejections, conditions related to incorrect proliferation or incorrect migration of smooth muscle cells, tumours and cancers, cutaneous stress, chronic inflammatory diseases, in particular rheumatoid arthritis, and pulmonary inflammatory diseases.

13. Use of a compound of formula (I) according to Claim 12, where the peripheral neuropathies are chosen from traumatic or ischaemic neuropathies or infectious, alcoholic, diabetic, medicinal or genetic neuropathies.

14. Use of a compound of formula (I) according to Claim 12, where the motor neuron conditions are chosen from spinal amyotrophies and amyotrophic lateral sclerosis.

15. Use of a compound of formula (I) according to Claim 12, where the neurodegenerative diseases of the central nervous system are chosen from strokes, cranial and medullar traumas, multiple sclerosis, Alzheimer's disease or Parkinson's disease.

## Patentansprüche

1. Verbindung der Formel (I), 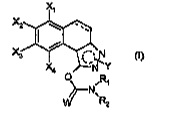 in der
W ein Sauerstoff- oder Schwefelatom bedeutet;
X₁, X₂, X₃ und X₄ jeweils unabhängig voneinander ein Wasserstoffatom oder ein Halogenatom oder eine Cyano-, C₁-C₆-Alkyl-, C₁-C₆-Fluoralkyl-, C₁-C₆-Alkoxy- oder C₁-C₆-Fluoralkoxygruppe bedeuten;
Y in (N2)- oder (N3)-Stellung steht;
wenn Y in (N2)-Stellung steht, Y eine C₁-C₆-Alkylgruppe, C₁-C₆-Fluoralkylgruppe, Arylgruppe mit zwischen 6 und 10 Kohlenstoffatomen oder Heteroarylgruppe mit 5 bis 6 Ringgliedern und zwischen 1 und 2 Heteroatomen bedeutet;
wenn Y in (N3)-Stellung steht, Y eine Arylgruppe oder Heteroarylgruppe bedeutet;
wobei die Aryl- oder Heteroarylgruppen gegebenenfalls durch ein(e) oder mehrere Atome oder Gruppen aus der Reihe Halogenatome, C₁-C₆-Alkyl-, C₁-C₆-Alkoxy-, C₁-C₆-Alkylthio-, C₁-C₆-Alkyl-S(O)-, C₁-C₆-Alkyl-S(O)₂-, C₁-C₆-Fluoralkylgruppe substituiert sind; die Bindung in C4-C5-Stellung doppelt oder einfach ist;
R₁ und R₂ jeweils unabhängig voneinander eine Arylgruppe mit zwischen 6 und 10 Kohlenstoffatomen, Benzyl oder C₁-C₆-Alkyl bedeuten; oder R₁ und R₂ mit dem Stickstoffatom, das sie trägt, einen Heterocyclus mit 4 bis 7 Ringgliedern und einem Stickstoffatom und gegebenenfalls einem weiteren Heteroatom bilden, der gegebenenfalls durch ein oder mehrere C₁-C₆-Alkyl- oder Benzylgruppen substituiert ist;
als Base oder Säureadditionssalz sowie als Hydrat oder Solvat.

2. Verbindung der Formel (I) nach Anspruch 1, **dadurch gekennzeichnet, daß**
W ein Sauerstoff- oder Schwefelatom bedeutet;
X₁, X₂ und X₃ jeweils unabhängig voneinander ein Wasserstoffatom oder ein Halogenatom oder eine Cyano-, C₁-C₆-Alkyl- oder C₁-C₆-Alkoxygruppe bedeuten;
X₄ ein Wasserstoffatom bedeutet;
Y in (N2)- oder (N3)-Stellung steht;
wenn Y in (N2)-Stellung steht, Y eine C₁-C₆-Alkylgruppe, C₁-C₆-Fluoralkylgruppe, Arylgruppe mit zwischen 6 und 10 Kohlenstoffatomen oder Heteroarylgruppe mit 5 bis 6 Ringgliedern und zwischen 1 und 2 Heteroatomen bedeutet;
wenn Y in (N3)-Stellung steht, Y eine Arylgruppe oder Heteroarylgruppe bedeutet;
wobei die Aryl- oder Heteroarylgruppen gegebenenfalls durch ein(e) oder mehrere Atome oder Gruppen aus der Reihe Halogenatome, C₁-C₆-Alkyl- und C₁-C₆-Alkoxygruppe substituiert sind;
die Bindung in C4-C5-Stellung doppelt oder einfach ist;
R₁ und R₂ jeweils unabhängig voneinander eine Arylgruppe mit zwischen 6 und 10 Kohlenstoffatomen oder C₁-C₆-Alkyl bedeuten; oder R₁ und R₂ mit dem Stickstoffatom, das sie trägt, einen Heterocyclus mit 4 bis 7 Ringgliedern und einem Stickstoffatom und gegebenenfalls einem weiteren Heteroatom bilden, der gegebenenfalls durch ein oder zwei C₁-C₆-Alkylgruppen substituiert ist; als Base oder Säureadditionssalz sowie als Hydrat oder Solvat.

3. Verbindung der Formel (I) nach Anspruch 1 oder 2,
**dadurch gekennzeichnet, daß**
W ein Sauerstoff- oder Schwefelatom bedeutet;
X₁, X₂ und X₃ jeweils unabhängig voneinander ein Wasserstoffatom oder ein Halogenatom oder eine C₁-C₆-Alkyl- oder C₁-C₆-Alkoxygruppe bedeuten;
X₄ ein Wasserstoffatom bedeutet;
Y in (N2)- oder (N3)-Stellung steht und eine Arylgruppe mit zwischen 6 und 10 Kohlenstoffatomen oder eine Heteroarylgruppe mit 5 bis 6 Ringgliedern und zwischen 1 und 2 Heteroatomen bedeutet;
wobei die Aryl- oder Heteroarylgruppen gegebenenfalls durch ein(e) oder mehrere Atome oder Gruppen aus der Reihe Halogenatome, C₁-C₆-Alkyl- und C₁-C₆-Alkoxygruppe substituiert sind;
die Bindung in C4-C5-Stellung doppelt oder einfach ist;
R₁ und R₂ jeweils unabhängig voneinander eine Arylgruppe mit zwischen 6 und 10 Kohlenstoffatomen oder C₁-C₆-Alkyl bedeuten; oder R₁ und R₂ mit dem Stickstoffatom, das sie trägt, einen Heterocyclus mit 4 bis 7 Ringgliedern und einem Stickstoffatom und gegebenenfalls einem weiteren Heteroatom bilden, der gegebenenfalls durch ein oder zwei C₁-C₆-Alkylgruppen substituiert ist;
als Base oder Säureadditionssalz sowie als Hydrat oder Solvat.

4. Verbindung der Formel (I) nach einem der Ansprüche 1 bis 3, **dadurch gekennzeichnet, daß**
W ein Sauerstoff- oder Schwefelatom bedeutet;
X_{1,} X₂ und X₃ jeweils unabhängig voneinander ein Wasserstoffatom oder ein Halogenatom oder eine C₁-C₆-Alkyl- oder C₁-C₆-Alkoxygruppe bedeuten;
X₄ ein Wasserstoffatom bedeutet;
Y in (N3)-Stellung steht und eine Arylgruppe mit zwischen 6 und 10 Kohlenstoffatomen oder eine Heteroarylgruppe mit 5 bis 6 Ringgliedern und zwischen 1 und 2 Heteroatomen bedeutet;
wobei die Aryl- oder Heteroarylgruppen gegebenenfalls durch ein(e) oder mehrere Atome oder Gruppen aus der Reihe Halogenatome, C₁-C₆-Alkyl- und C₁-C₆-Alkoxygruppe substituiert sind;
die Bindung in C4-C5-Stellung doppelt oder einfach ist;
R₁ und R₂ jeweils unabhängig voneinander eine Arylgruppe mit zwischen 6 und 10 Kohlenstoffatomen oder C₁-C₆-Alkyl bedeuten; oder R₁ und R₂ mit dem Stickstoffatom, das sie trägt, einen Heterocyclus mit 4 bis 7 Ringgliedern und einem Stickstoffatom und gegebenenfalls einem weiteren Heteroatom bilden, der gegebenenfalls durch ein oder zwei C₁-C₆-Alkylgruppen substituiert ist;
als Base oder Säureadditionssalz sowie als Hydrat oder Solvat.

5. Verbindung der Formel (I) nach einem der Ansprüche 1 bis 4, **dadurch gekennzeichnet, daß** sie aus der folgenden Reihe stammt:
7-Fluor-2-(4-fluorphenyl)-4,5-dihydro-2*H-*benzo[e]indazol-1-yl-*N,N*-diethylcarbamat;
7-Fluor-2-(4-fluorphenyl)-2*H*-benzo[e]indazol-1-yl-*N,N*-diethylcarbamat;
7-Chlor-2-(pyridin-4-yl)-4,5-dihydro-2*H-*benzo[e]indazol-1-yl-*N,N*-diethylcarbamathydrochlorid;
7-Chlor-3-(pyridin-4-yl)-4,5-dihydro-3*H-*benzo[e]indazol-1-yl-*N,N*-diethylcarbamathydrochlorid;
8-Methoxy-3-(pyridin-4-yl)-4,5-dihydro-3*H-*benzo[e]indazol-1-yl-*N,N*-diethylcarbamathydrochlorid;
2-(4-Methylphenyl)-4,5-dihydro-2*H*-benzo[e]indazol-1-yl-*N,N*-dimethylcarbamat;
2-(4-Chlorphenyl)-4,5-dihydro-2*H*-benzo[e]indazol-1-yl-*N,N*-diethylcarbamat;
2-(3-Chlorphenyl)-4,5-dihydro-2*H*-benzo[e]indazol-1-yl-morpholinyl-4-carboxylat;
2-(3-Chlorphenyl)-4,5-dihydro-2*H*-benzo[e]indazol-1-yl-*N,N*-dimethylcarbamat;
2-(3-Chlorphenyl)-4,5-dihydro-2*H*-benzo[e]indazol-1-yl-*N,N*-diethylcarbamat;
2-(3-Chlorphenyl)-4,5-dihydro-2*H*-benzo[e]indazol-1-yl-pyrrolidinyl-1-carboxylat;
2-(3-Chlorphenyl)-4,5-dihydro-2*H*-benzo[e]indazol-1-yl-*N,N*-diisopropylcarbamat;
2-Phenyl-4,5-dihydro-2*H*-benzo[e]indazol-1-yl-*N,N-*diethylcarbamat;
7-Chlor-2-phenyl-4,5-dihydro-2*H*-benzo[e]indazol-1-yl-*N,N*-diethylcarbamat;
2-(3-Chlorphenyl)-4,5-dihydro-2*H*-benzo[e]indazol-1-yl-*N*-methyl-*N*-phenylcarbamat;
2-(2-Chlorphenyl)-4,5-dihydro-2*H*-benzo[e]indazol-1-yl-*N,N*-diethylcarbamat;
2-Ethyl-4,5-dihydro-2*H*-benzo[e]indazol-1-yl-*N,N-*diethylcarbamat;
2-Ethyl-4,5-dihydro-2*H*-benzo[e]indazol-1-yl-N-methyl-*N*-phenylcarbamat;
2-(2,2',2"-Trifluorethyl)-4,5-dihydro-2*H-*benzo[e]indazol-1-yl-*N*-methyl-*N*-phenylcarbamat;
2-(4-Fluorphenyl)-4,5-dihydro-2*H*-benzo[e]indazol-1-yl-*N,N*-diethylcarbamat;
2-(4-Fluorphenyl)-4,5-dihydro-2*H*-benzo[e]indazol-1-yl-*N,N*-diisopropylcarbamat;
2-(4-Fluorphenyl)-4,5-dihydro-2*H*-benza[e]indazol-1-yl-*N*-methyl-*N*-phenylcarbamat;
2-(4-Fluorphenyl)-4,5-dihydro-2*H*-benzo[e]indazol-1-yl-pyrrolidinyl-1-carboxylat:
2-(4-Fluorphenyl)-4,5-dihydro-2*H*-benzo[e]indazol-1-yl-piperidinyl-1-carboxylat;
2-(4-Fluorphenyl)-4,5-dihydro-2*H*-benzo[e]indazol-1-yl-morpholinyl-4-carboxylat;
2-(4-Chlorphenyl)-4,5-dihydro-2*H*-benzo[e]indazol-1-yl-*N,N*-dimethylcarbamat;
2-(4-Chlorphenyl)-4,5-dihydro-2*H*-benzo[e]indazol-1-yl-*N,N*-diisopropylcarbamat;
2-(4-Chlorphenyl)-4,5-dihydro-2*H*-benzo[e]indazol-1-yl-*N*-methyl-*N*-phenylcarbamat;
2-(4-Chlorphenyl)-4,5-dihydro-2*H*-benzo[e]indazol-1-yl-pyrrolidinyl-1-carboxylat;
2-(4-Chlorphenyl)-4,5-dihydro-2*H*-benzo[e]indazol-1-yl-piperidinyl-1-carboxylat;
2-(4-Chlorphenyl)-4,5-dihydro-2*H*-benzo[e]indazol-1-yl-morpholinyl-4-carboxylat;
2-(4-Chlorphenyl)-4,5-dihydro-2*H*-benzo[e]indazol-1-yl-4-methyl-piperazinyl-1-carboxylat;
2-(4-Fluorphenyl)-4,5-dihydro-2*H*-benzo[e]indazol-1-yl-*N,N*-dimethylcarbamat;
2-(4-Fluorphenyl)-4,5-dihydro-2*H*-benzo[e]indazol-1-yl-4-methyl-piperazinyl-1-carboxylat;
2-Phenyl-4,5-dihydro-2*H*-benzo[e]indazol-1-yl-*N,N-*dimethylcarbamat;
2-Phenyl-4,5-dihydro-2*H*-benzo[e]indazol-1-yl-*N,N-*diisopropylcarbamat;
2-Phenyl-4,5-dihydro-2*H*-benzo[e]indazol-1-yl-*N-*methyl-*N*-phenylcarbamat;
2-Phenyl-4,5-dihydro-2*H*-benzo[e]indazol-1-yl-pyrrolidinyl-1-carboxylat;
7-Chlor-2-(4-fluorphenyl)-4,5-dihydro-2*H-*benzo[e]indazol-1-yl-*N,N*-diethylcarbamat;
2-(4-Fluorphenyl)-4,5-dihydro-2*H*-benzo[e]indazol-1-yl-*N*-*N*-propylcarbamat;
2-(3-Chlor-4-methylphenyl)-4,5-dihydro-2*H-*benzo[e]indazol-1-yl-*N,N*-diethylcarbamat;
2-(3-Chlor-4-fluorphenyl)-4,5-dihydro-2*H-*benzo[e]indazol-1-yl-*N,N*-diethylcarbamat;
7-Chlor-2-(3-chlor-4-methylphenyl)-4,5-dihydro-2*H-*benzo[e]indazol-1-yl-*N,N*-diethylcarbamat;
7-Chlor-2-(2,4-difluorphenyl)-4,5-dihydro-2*H-*benzo[e]indazol-1-yl-*N,N*-diethylcarbamat;
2-(Pyridin-4-yl)-4,5-dihydro-2*H*-benzo[e]indazol-1-yl-*N,N*-diethylcarbamat-hydrochlorid;
7-Chlor-2-(pyridin-2-yl)-4,5-dihydro-2*H-*benzo[e]indazol-1-yl-*N,N*-diethylcarbamat;
7-Methoxy-2-(4-fluorphenyl)-4,5-dihydro-2*H-*benzo[e]indazol-1-yl-*N,N*-diethylcarbamat;
7-Brom-2-(4-fluorphenyl)-4,5-dihydro-2*H-*benzo[e]indazol-1-yl-*N,N*-diethylcarbamat;
7-Chlor-2-(3-chlor-4-fluorphenyl)-4,5-dihydro-2*H-*benzo[e]indazol-1-yl-*N,N*-diethylcarbamat;
7-Chlor-2-methyl-4,5-dihydro-2*H*-benzo[e]indazol-1-yl-*N,N*-diethylcarbamat;
2-(3-Chlorphenyl)-2*H*-benzo[e]indazol-1-yl-*N,N-*diethylcarbamat;
2-(4-Fluorphenyl)-2*H*-benzo[e]indazol-1-yl-*N,N-*diethylcarbamat;
2-(4-Fluorphenyl)-2*H*-benzo[e]indazol-1-yl-*N-*methyl-*N*-propylcarbamat;
2-(3-Chlor-4-fluorphenyl)-2*H*-benzo[e]indazol-1-yl-*N,N*-diethylcarbamat;
2-(3-Chlor-4-methylphenyl)-2*H*-benzo[e]indazol-1-yl-*N,N*-diethylcarbamat;
7-Chlor-2-(4-fluorphenyl)-2*H*-benzo[e]indazol-1-yl-*N,N*-diethylcarbamat;
7-Chlor-2-(3-chlor-4-fluorphenyl)-2*H-*benzo[e]indazol-1-yl-*N,N*-diethylcarbamat;
7-Chlor-2-(3-chlor-4-methylphenyl)-2*H-*benzo[e]indazol-1-yl-*N,N*-diethylcarbamat;
7-Chlor-2-(2,4-difluorphenyl)-2*H*-benzo[e]indazol-1-yl-*N,N*-diethylcarbamat;
7-Methoxy-3-(pyridin-4-yl)-4,5-dihydro-3*H-*benzo[e]indazol-1-yl-N,N-diethylcarbamat-hydrochlorid;
7-Methyl-3-(pyridin-4-yl)-4,5-dihydro-3*H-*benzo[e]indazol-1-yl-N,N-diethylcarbamat-hydrochlorid;
7-Chlor-3-(pyridin-3-yl)-4,5-dihydro-3*H-*benzo[e]indazol-1-yl-N,N-diethylcarbamat-hydrochlorid;
7-Chlor-2-(pyridin-3-yl)-4,5-dihydro-2*H-*benzo[e]indazol-1-yl-N,N-diethylcarbamat-hydrochlorid;
7-Fluor-2-(pyridin-4-yl)-4,5-dihydro-2*H-*benzo[e]indazol-1-yl-N,N-diethylcarbamat-hydrochlorid;
7-Fluor-3-(pyridin-4-yl)-4,5-dihydro-3*H-*benzo[e]indazol-1-yl-N,N-diethylcarbamat-hydrochlorid;
7-Chlor-3-(4-fluorphenyl)-4,5-dihydro-3*H-*benzo[e]indazol-1-yl-N,N-diethylcarbamat;
7-Chlor-3-(pyridin-2-yl)-4,5-dihydro-3*H-*benzo[e]indazol-1-yl-N,N-diethylcarbamat-hydrochlorid;
7-Chlor-2-(3-fluorphenyl)-4,5-dihydro-2*H-*benzo[e]indazol-1-yl-N,N-diethylcarbamat;
7-Chlor-3-(3-fluorphenyl)-4,5-dihydro-3*H-*benzo[e]indazol-1-yl-N,N-diethylcarbamat;
7-Brom-2-(pyridin-4-yl)-4,5-dihydro-2*H-*benzo[e]indazol-1-yl-N,N-diethylcarbamat-hydrochlorid;
7-Brom-3-(pyridin-4-yl)-4,5-dihydro-3*H-*benzo[e]indazol-1-yl-N,N-diethylcarbamat-hydrochlorid;
7-Chlor-3-(4-methylphenyl)-4,5-dihydro-3*H-*benzo[e]indazol-1-yl-N,N-diethylcarbamat;
7-Chlor-2-(4-methylphenyl)-4,5-dihydro-2*H-*benzo[e]indazol-1-yl-N,N-diethylcarbamat;
7-Chlor-2-(2-fluorphenyl)-4,5-dihydro-2*H-*benzo[e]indazol-1-yl-N,N-diethylcarbamat;
7-Chlor-3-(4-chlorphenyl)-4,5-dihydro-3*H-*benzo[e]indazol-1-yl-N,N-diethylcarbamat;
7-Chlor-2-(4-chlorphenyl)-4,5-dihydro-2*H-*benzo[e]indazol-1-yl-N,N-diethylcarbamat;
7-Chlor-2-(4-methoxyphenyl)-4,5-dihydro-2*H-*benzo[e]indazol-1-yl-N,N-diethylcarbamat;
7-Chlor-3-(4-methoxyphenyl)-4,5-dihydro-3*H-*benzo[e]indazol-1-yl-N,N-diethylcarbamat;
7-Chlor-3-(3-fluorphenyl)-3*H*-benzo[e]indazol-1-yl-N,N-diethylcarbamat;
7-Chlor-3-(pyridin-4-yl)-4,5-dihydro-3*H-*benzo[e]indazol-1-yl-*N*-methyl-*N*-phenylcarbamat;
7-Chlor-2-(pyridin-4-yl)-4,5-dihydro-2*H-*benzo[e]indazol-1-yl-N,N-diisopropylcarbamat;
7-Chlor-4,5-dihydro-3-(pyridin-4-yl)-3*H-*benzo[e]indazol-1-yl-N,N-diisopropylcarbamat-hydrochlorid;
6-Methoxy-3-(pyridin-4-yl)-4,5-dihydro-3*H-*benzo[e]indazol-1-yl-N,N-diethylcarbamat-hydrochlorid;
7-Chlor-3-(pyridin-4-yl)-3*H*-benzo[e]indazol-1-yl-*N,N*-diisopropylcarbamat-hydrochlorid;
7-Chlor-2-(pyridin-4-yl)-4,5-dihydro-2*H-*benzo[e]indazol-1-yl-piperidinyl-1-carboxylat-hydrochlorid;
7-Chlor-3-(pyridin-4-yl)-4,5-dihydro-3*H-*benzo[e]indazol-1-yl-piperidinyl-1-carboxylat-hydrochlorid;
7-Chlor-2-(pyridin-3-yl)-4,5-dihydro-2*H-*benzo[e]indazol-1-yl-*N,N*-diisopropylcarbamat-hydrochlorid;
7-Chlor-3-(pyridin-3-yl)-4,5-dihydro-3*H-*benzo[e]indazol-1-yl-*N,N*-diisopropylcarbamat-hydrochlorid;
7-Chlor-3-(pyridin-4-yl)-4,5-dihydro-3*H-*benzo[e]indazol-1-yl-*N,N*-diisopropylcarbamat-hydrochlorid;
7-Chlor-3-(pyridin-4-yl)-4,5-dihydro-3*H-*benzo[e]indazol-1-yl-cis-2,6-dimethylpiperidinyl-1-carboxylat-hydrochlorid;
7-Chlor-2-(pyridin-4-yl)-4,5-dihydro-2*H-*benzo[e]indazol-1-yl-*N*-methyl-*N*-tertbutylcarbamat-hydrochlorid;
7-Chlor-3-(pyridin-3-yl)-3*H*-benzo[e]indazol-1-yl-*N,N*-diisopropylcarbamat-hydrochlorid;
7-Chlor-3-(pyridin-2-yl)-4,5-dihydro-3*H-*benzo[e]indazol-1-yl-*N,N*-diisopropylcarbamat;
7-Fluor-3-(pyridin-4-yl)-4,5-dihydro-3*H-*benzo[e]indazol-1-yl-*N,N*-diisopropylcarbamat-hydrochlorid;
7-Chlor-3-(pyrimidin-2-yl)-4,5-dihydro-3*H-*benzo[e]indazol-1-yl-*N,N*-diisopropylcarbamat;
7-Chlor-2-(pyrazin-2-yl)-4,5-dihydro-2*H-*benzo[e]indazol-1-yl-*N,N*-diisopropylcarbamat;
7-Fluor-3-(pyridin-4-yl)-3*H*-benzo[e]indazol-1-yl-*N,N*-diisopropylcarbamat-hydrochlorid;
7-Chlor-3-(pyridin-2-yl)-3*H*-benzo[e]indazol-1-yl-*N,N*-diisopropylcarbamat;
7-Chlor-3-(pyridin-4-yl)-4,5-dihydro-3*H-*benzo[e]indazol-1-yl-*N*-methyl-*N*-tertbutylcarbamat-hydrochlorid;
7-Chlor-3-(pyridin-4-yl)-3*H*-benzo[e]indazol-1-yl-*N,N*-diethylcarbamat-hydrochlorid;
7-Chlor-3-(pyridin-4-yl)-3*H*-benzo[e]indazol-1-yl-*N*-methyl-*N*-phenylcarbamat-hydrochlorid;

6. Verfahren zur Herstellung einer Verbindung der Formel (I) nach einem der Ansprüche 1 bis 5,
**dadurch gekennzeichnet, daß** man
eine Mischung, die aus Stellungsisomeren der allgemeinen Formeln (Va) und (Vb) besteht, 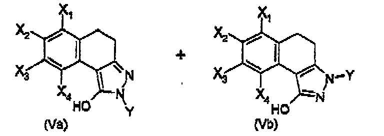 wobei X₁, X₂, X₃, X₄ und Y wie in der allgemeinen Formel (I) nach Anspruch 1 definiert sind und
wobei die Gruppe Y in (N2)- bzw. (N3)-Stellung des Pyrazolrings steht,
mit einem Carbamoylchloridderivat der allgemeinen Formel CIC(W)NR₁R₂, in der W, R₁ und R₂ wie in der allgemeinen Formel (I) nach Anspruch 1 definiert sind, in Gegenwart einer Base umsetzt, wodurch man nach Trennung zu den Verbindungen der allgemeinen Formeln (Ia) und (Ib) gelangt, 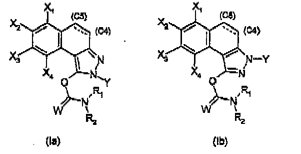 wobei gegebenenfalls die Einfachbindung in C4-C5-Stellung anschließend dehydriert wird, um zu einer Doppelbindung zu gelangen.

7. Verfahren zur Herstellung einer Verbindung der Formel (Ia), 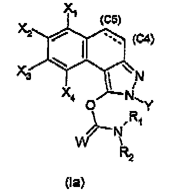 in der
W ein Sauerstoff- oder Schwefelatom bedeutet;
X₁, X₂, X₃ und X₄ jeweils unabhängig voneinander ein Wasserstoffatom oder ein Halogenatom oder eine Cyano-, C₁-C₆-Alkyl-, C₁-C₆-Fluoralkyl-, C₁-C₆-Alkoxy- oder C₁-C₆-Fluoralkoxygruppe bedeuten;
Y in (N2)-Stellung steht und eine C₁-C₆-Alkylgruppe, C₁-C₆-Fluoralkylgruppe, Arylgruppe mit zwischen 6 und 10 Kohlenstoffatomen oder Heteroarylgruppe mit 5 bis 6 Ringgliedern und zwischen 1 und 2 Heteroatomen bedeutet,
wobei die Aryl- oder Heteroarylgruppen gegebenenfalls durch ein(e) oder mehrere Atome oder Gruppen aus der Reihe Halogenatome, C₁-C₆-Alkyl-, C₁-C₆-Alkoxy-, C₁-C₆-Alkylthio-, C₁-C₆-Alkyl-S(O)-, C₁-C₆-Alkyl-S(O)₂-, C₁-C₆-Fluoralkylgruppe substituiert sind;
die Bindung in C4-C5-Stellung doppelt oder einfach ist;
R₁ und R₂ jeweils unabhängig voneinander eine Arylgruppe mit zwischen 6 und 10 Kohlenstoffatomen, Benzyl oder C₁-C₆-Alkyl bedeuten; oder R₁ und R₂ mit dem Stickstoffatom, das sie trägt, einen Heterocyclus mit 4 bis 7 Ringgliedern und einem Stickstoffatom und gegebenenfalls einem weiteren Heteroatom bilden, der gegebenenfalls durch ein oder mehrere C₁-C₆-Alkyl- oder Benzylgruppen substituiert ist;
**dadurch gekennzeichnet, daß** man die Verbindung der allgemeinen Formel (Va), 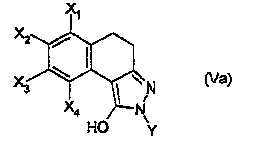 in der X₁, X₂, X₃, X₄ und Y wie in der allgemeinen Formel (Ia) oben definiert sind,
mit einem Carbamoylchloridderivat der allgemeinen Formel CIC(W)NR₁R₂, in der W, R₁ und R₂ wie in der allgemeinen Formel (Ia) oben definiert sind, in Gegenwart einer Base umsetzt,
wobei die Einfachbindung in C4-C5-Stellung gegebenenfalls anschließend dehydriert wird, um eine Doppelbindung zu bilden.

8. Verbindung der Formel (Va), 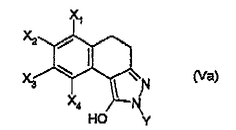 in der
X₁, X₂, X₃ und X₄ jeweils unabhängig voneinander ein Wasserstoffatom oder ein Halogenatom oder eine Cyano-, C₁-C₆-Alkyl-, C₁-C₆-Fluoralkyl-, C₁-C₆-Alkoxy- oder C₁-C₆-Fluoralkoxygruppe bedeuten;
Y eine C₁-C₆-Alkylgruppe, C₁-C₆-Fluoralkylgruppe, Arylgruppe mit zwischen 6 und 10 Kohlenstoffatomen oder Heteroarylgruppe mit 5 bis 6 Ringgliedern und zwischen 1 und 2 Heteroatomen bedeutet,
wobei die Aryl- oder Heteroarylgruppen gegebenenfalls durch ein(e) oder mehrere Atome oder Gruppen aus der Reihe Halogenatome, C₁-C₆-Alkyl-, C₁-C₆-Alkoxy-, C₁-C₆-Alkylthio-, C₁-C₆-Alkyl-S(O)-, C₁-C₆-Alkyl-S(O)₂-, C₁-C₆-Fluoralkylgruppe substituiert sind.

9. Verbindung der Formel (Vb), 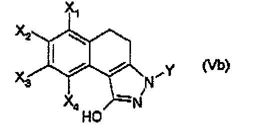 in der
X₁, X₂, X₃ und X₄ jeweils unabhängig voneinander ein Wasserstoffatom oder ein Halogenatom oder eine Cyano-, C₁-C₆-Alkyl-, C₁-C₆-Fluoralkyl-, C₁-C₆-Alkoxy- oder C₁-C₆-Fluoralkoxygruppe bedeuten;
Y eine Arylgruppe mit zwischen 6 und 10 Kohlenstoffatomen oder eine Heteroarylgruppe mit 5 bis 6 Ringgliedern und zwischen 1 und 2 Heteroatomen bedeutet,
wobei die Aryl- oder Heteroarylgruppen gegebenenfalls durch ein(e) oder mehrere Atome oder Gruppen aus der Reihe Halogenatome, C₁-C₆-Alkyl-, C₁-C₆-Alkoxy-, C₁-C₆-Alkylthio-, C₁-C₆-Alkyl-S(O)-, C₁-C₆-Alkyl-S(O)₂-, C₁-C₆-Fluoralkylgruppe substituiert sind.

10. Arzneimittel, **dadurch gekennzeichnet, daß** es eine Verbindung der Formel (I) nach einem der Ansprüche 1 bis 5 oder ein Säureadditionssalz dieser Verbindung mit einer pharmazeutisch unbedenklichen Säure oder auch ein Hydrat oder Solvat der Verbindung der Formel (I) enthält.

11. Pharmazeutische Zusammensetzung, **dadurch gekennzeichnet, daß** sie eine Verbindung der Formel (I) nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Grundstoff enthält.

12. Verwendung einer Verbindung der Formel (I) nach einem der Ansprüche 1 bis 5 zur Herstellung eines Arzneimittels für die Vorbeugung oder Behandlung von peripheren Neuropathien, Krankheiten der motorischen Nervenzellen, neurodegenerativen Krankheiten des Zentralnervensystems, Angstzuständen, Epilepsie, Schlafstörungen, akuter oder chronischer Niereninsuffizienz, Glomerulonephritis, diabetesbedingter Nierenschädigung, Herzischämie und -insuffizienz, Myocardinfarkt, Ischämie der unteren Gliedmaßen, koronarem Gefäßkrampf, Angina pectoris, mit den Herzklappen assoziierten Pathologien, entzündlichen Herzkrankheiten, Nebenwirkungen aufgrund von kardiotoxischen Medikamenten oder im Anschluß an Herzchirurgie, Arteriosklerose und ihre Thromboembolie-Komplikationen, Restenose, Transplantatabstoßungen, Erkrankungen, im Zusammenhang mit inkorrekter Proliferation oder Wanderung von glatten Muskelzellen, Tumoren und Karzinomen, Hautbelastungen, chronischen Entzündungskrankheiten, insbesondere rheumatoider Polyarthritis, und entzündlichen Lungenkrankheiten.

13. Verwendung einer Verbindung der Formel (I) nach Anspruch 12, wo die peripheren Neuropathien aus der folgenden Gruppe stammen: traumatische oder ischämische Neuropathien, infektiöse Neuropathien, alkoholische Neuropathien, diabetische Neuropathien, medikamentöse Neuropathien oder genetische Neuropathien.

14. Verwendung einer Verbindung der Formel (I) nach Anspruch 12, wo die Krankheiten der motorischen Nervenzellen aus den Wirbelsäulenamyotrophien und amyotrophischer Lateralsklerose stammen.

15. Verwendung einer Verbindung der Formel (I) nach Anspruch 12, wo die neurodegenerativen Krankheiten des Zentralnervensystems aus den folgenden ausgewählt sind: Hirngefäßverletzungen, Schädel- und Medullatraumen, Multiple Sklerose, Morbus Alzheimer oder Morbus Parkinson.
